# EUROPEAN PATENT SPECIFICATION

(11) **EP 0 319 073 B1**
(45) Date of publication and mention of the grant of the patent: **23.08.1995**
(21) Application number: 88202618.0
(22) Date of filing: 22.11.1988
(51) Int. Cl.: C07D 487/08, A61K 31/55

(54) **Cyclo-octane neuroprotective agents**
Neuroschützende Cyclooctanmittel
Agents neuroprotecteurs dérivés du cyclooctane

(30) Priority: 30.11.1987 GB 8727989
(43) Date of publication of application: 07.06.1989
(73) Proprietor: MERCK SHARP & DOHME LTD., Hoddesdon Hertfordshire EN11 9NU (GB)
(72) Inventor: Baker, Raymond, Much Hadham Hertfordshire (GB); Carling, William R., Bishops Stortford Hertfordshire (GB); James, Kim, Welwyn Garden City Hertfordshire (GB); Leeson, Paul D., Cambridge Cambridgeshire (GB)
(74) Representative: Cole, William Gwyn

(56) References cited:
- EP-A- 0 220 339
- DE-A- 2 026 486
- GB-A- 2 004 550
- JOURNAL OF THE CHEMICAL SOCIETY. PERKIN TRANSACTIONS 1, no. 17, 1972, pages 2205-2213, The Chemical Society, London, GB; J.W. BASTABLE et al.: "Transannular cyclisation of cyclo-olefinic N-chloro-amines. Synthesis of azabicyclic compounds"
- JOURNAL OF THE AMERICAN CHEMICAL SOCIETY, vol. 108, no. 24, November 1986, pages 7864-7865, American Chemical Society, Washington, DC, US; D.B. KANNE et al.: "Synthesis of the first highly potent bridged nicotinoid. 9-Azabicyclo[4.2.1]nona[2,3-c]pyridine (Pyrido[3,4-b]homotropane)"

## Description

This invention relates to a class of cyclooctenimine derivatives which are specific antagonists of N-methyl-D-aspartate (NMDA) receptors and are therefore useful in the treatment and/or prevention of neurodegenerative disorders arising as a consequence of such pathological conditions as stroke, hypoglycaemia, cerebral palsy, transient cerebral ischaemic attack, cerebral ischaemia during cardiac pulmonary surgery or cardiac arrest, perinatal asphyxia, epilepsy, Huntington's chorea, Alzheimer's disease, Olivo-ponto-cerebellar atrophy, anoxia such as from drowning, spinal cord injury and poisoning by exogenous NMDA poisons. The compounds are also useful as anticonvulsant agents.

Certain dibenzocyclooctenimines are disclosed in U.S. Patent No. 4,414,154, and British Patent No. 2,004,550, and the use of those compounds in the treatment of neurodegenerative diseases is described in published European Patent Application No. 230,370. The prior art compounds are dibenzo[a,d]cyclooctenimine derivatives.

The cyclooctenimines of this invention comprise either a dibenzo[a,e]cyclooctenimine moiety, which represents a novel ring system, or they possess no fused benzo ring. The cyclooctenimines of this invention, including dibenzo derivatives thereof, have been found to be highly potent non-competitive centrally acting selective NMDA receptor antagonists.

Accordingly, the present invention provides a compound of formula I: 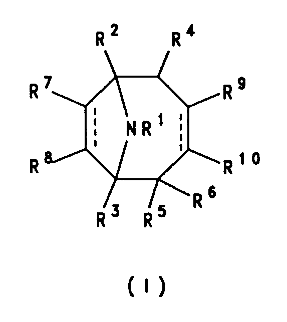
or a salt thereof, wherein:
the dotted lines represent optional double bonds;
R¹ represents hydrogen, hydroxy, C₂₋₆ alkenyl, alkyl containing from 1 to 6 carbon atoms, aminoalkyl containing from 1 to 6 carbon atoms or hydroxyalkyl containing from 1 to 6 carbon atoms;
R², R³, R⁴, R⁵ and R⁶ independently represent hydrogen, hydroxy, fluoro, C₂₋₆ alkenyl, aryl, alkyl containing from 1 to 6 carbon atoms, or alkyl containing from 1 to 6 carbon atoms substituted with aryl, amino, hydroxy, carboxy or fluoro; and
R⁷, R⁸, R⁹ and R¹⁰ independently represent hydrogen, hydrocarbon having up to 18 carbon atoms, or a heterocyclic group, which hydrocarbon or heterocyclic groups are optionally substituted with a group selected from halogen, C₁₋₆ alkyl, aryl, arylalkyl, C₁₋₆ alkoxy, halo(C₁₋₆)alkyl, hydroxy, amino, nitro, carboxy, C₁₋₆ alkoxycarbonyl, C₁₋₆ alkoxycarbonyl-(C₁₋₆)alkyl, C₁₋₆ alkylcarbonyloxy and C₁₋₆ alkylcarbonyl groups provided that R⁷, R⁸, R⁹ and R¹⁰ are not simultaneously hydrogen; or
R⁷ and R⁸ and/or R⁹ and R¹⁰ may complete a saturated or unsaturated C₄₋₉ hydrocarbon or heterocyclic ring.

The term 'hydrocarbon having up to 18 carbon atoms' includes groups having suitably up to 10 carbon atoms, conveniently up to 6 carbon atoms. Suitable hydrocarbon groups include C₁₋₆ alkyl, C₂₋₆ alkenyl, C₂₋₆ alkynyl, C₃₋₇ cycloalkyl, C₃₋₇ cycloalkyl(C₁₋₆)alkyl, C₅₋₇ cycloalkenyl, C₅₋₇ cycloalkenyl(C₁₋₆)alkyl, aryl and aryl(C₁₋₆)alkyl.

Suitable alkyl groups include straight and branched chain alkyl groups containing from 1 to 6 carbon atoms, such as methyl, ethyl, propyl and butyl.

When used herein the term 'aryl' includes optionally substituted phenyl and naphthyl.

Suitable alkenyl groups are C₂₋₆ alkenyl, such as ethenyl.

The term 'heterocyclic' includes single or fused rings comprising up to four hetero atoms in the ring selected from oxygen, nitrogen and sulphur. Suitable heterocyclic groups include pyridyl, thienyl, furyl, indolyl, benzothienyl, benzofuryl and quinolinyl.

Any of the hydrocarbon or heterocyclic groups may, as indicated above, be substituted with a group selected from halogen, C₁₋₆ alkyl, aryl, arylalkyl, C₁₋₆ alkoxy, halo(C₁₋₆)alkyl, hydroxy, amino, nitro, carboxy, C₁₋₆ alkoxycarbonyl, C₁₋₆ alkoxycarbonyl(C₁₋₆)alkyl, C₁₋₆ alkylcarbonyloxy and C₁₋₆ alkylcarbonyl groups. Preferred substituent groups include halogen, hydroxy, C₁₋₆ alkyl and C₁₋₆ alkoxy.

Suitable acid addition salts of compounds of this invention include pharmaceutically acceptable inorganic salts such as sulphate, nitrate, phosphate, borate, hydrochloride and hydrobromide, and pharmaceutically acceptable organic acid addition salts such as acetate, tartrate, maleate, citrate, succinate, benzoate, ascorbate, methanesulphonate, α-ketoglutarate, α-glycerophosphate and glucose-1-phosphate. Preferably the acid addition salt is a hemisuccinate, hydrochloride, α-ketoglutarate, α-glycerophosphate, maleate or glucose-1-phosphate, in particular the hydrochloride or maleate salt.

The substituents R⁴, R⁵ and R⁶ may be present in the exo or endo configuration. The present invention includes all such isomers and mixtures thereof. In addition, when a compound of the invention has a chiral centre, all enantiomers and mixtures thereof, including racemic mixtures thereof, are included within the invention.

When R⁷ and R⁸ or R⁹ and R¹⁰ together represent the residue of a hydrocarbon ring, the ring may be saturated or unsaturated. The R⁷/R⁸ or R⁹/R¹⁰ carbon chain may comprise from 2 to 7 carbon atoms, preferably 3 or 4 carbon atoms, i.e. forming a fused 5- or 6-membered ring. Preferably R⁷ and R⁸ and also R⁹ and R¹⁰ complete optionally substituted benzo or cyclohexane rings.

Preferred values for the substituent R¹ are hydrogen and C₁₋₄ alkyl.

Preferably R² to R⁶ independently represent hydrogen, hydroxy, fluoro or C₁₋₆ alkyl, especially hydrogen, hydroxy or C₁₋₄ alkyl.

One subgroup of compounds of this invention is represented by formula II: 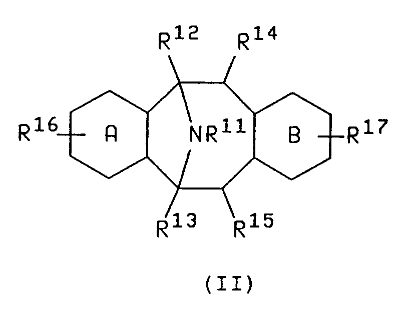
wherein rings A and B independently represent cyclohexane, cyclohexene, cyclohexadiene or benzene rings; R¹¹ is as defined above for R¹; and R¹² to R¹⁷ independently represent hydrogen, hydroxy, C₁₋₆ alkyl, aryl, arylalkyl, C₁₋₆ alkoxy or halogen.

When the bond common to ring A or ring B and the cyclooctane ring is saturated, each bridgehead position may be in either the exo or the endo configuration. This leads to a maximum of two cis and two trans isomers for each structure. The bridgehead positions are also chiral and therefore will lead to enantiomeric isomers.

Preferably rings A and B represent benzene rings and R¹⁶ and R¹⁷ independently represent hydrogen, methyl, hydroxy, methoxy, bromo or chloro. Preferably R¹³ is methyl.

Particular compounds of this invention include:
6-methyl-5,6,11,12-tetrahydrodibenzo[a,e]cycloocten-6,11-imine;
11-n-butyl-6-methyl-5,6,11,12-tetrahydrodibenzo[a,e]cycloocten-6,11-imine;
5,6-dimethyl-5,6,11,12-tetrahydrodibenzo[a,e]cycloocten-6,11-imine;
2-bromo-6-methyl-5,6,11,12-tetrahydrodibenzo[a,e]cycloocten-6,11-imine;
9-bromo-6-methyl-5,6,11,12-tetrahydrodibenzo[a,e]cycloocten-6,11-imine;
3,6-dimethyl-5,6,11,12-tetrahydrodibenzo[a,e]cycloocten-6,11-imine;
6,8-dimethyl-5,6,11,12-tetrahydrodibenzo[a,e]cycloocten-6,11-imine;
2-chloro-6-methyl-5,6,11,12-tetrahydrodibenzo[a,e]cycloocten-6,11-imine;
9-chloro-6-methyl-5,6,11,12-tetrahydrodibenzo[a,e]cycloocten-6,11-imine;
3-bromo-6-methyl-5,6,11,12-tetrahydrodibenzo[a,e]cycloocten-6,11-imine;
6-methyl-5,6,6a-exo,7,8,9,10,10a-exo,11,12-decahydrodibenzo[a,e]cycloocten-6,11-imine;
6-methyl-5,6,6a-endo,7,8,9,10,10a-endo,11,12-decahydrodibenzo[a,e]cycloocten-6,11-imine;
5,6,11,12-tetrahydrodibenzo[a,e,]cycloocten-6,11-imine;
6,11-dimethyl-5,6,11,12-tetrahydrodibenzo[a,e]cycloocten-6,11-imine;
2-methoxy-6-methyl-5,6,11,12-tetrahydrodibenzo[a,e]cycloocten-6,11-imine;
3-methoxy-6-methyl-5,6,11,12-tetrahydrodibenzo[a,e]cycloocten-6,11-imine;
2-hydroxy-6-methyl-5,6,11,12-tetrahydrodibenzo[a,e]cycloocten-6,11-imine;
3-hydroxy-6-methyl-5,6,11,12-tetrahydrodibenzo[a,e]cycloocten-6,11-imine;
12-exo-hydroxy-6-methyl-5,6,11,12-tetrahydrodibenzo[a,e]cycloocten-6,11-imine;
1-chloro-6-methyl-5,6,11,12-tetrahydrodibenzo[a,e]cycloocten-6,11-imine;
10-chloro-6-methyl-5,6,11,12-tetrahydrodibenzo[a,e]cycloocten-6,11-imine;
6-ethyl-5,6,11,12-tetrahydrodibenzo[a,e]cycloocten-6,11-imine;
8-bromo-6-methyl-5,6,11,12-tetrahydrodibenzo[a,e]cycloocten-6,11-imine;
11-hydroxy-6-methyl-5,6,11,12-tetrahydrodibenzo[a,e]cycloocten-6,11-imine;
6,13-dimethyl-5,6,11,12-tetrahydrodibenzo[a,e]cycloocten-6,11-imine;
and salts thereof.

Also included within the scope of the present invention are pharmaceutical compositions comprising the imines of this invention. Preferably these compositions are in unit dosage forms such as tablets, pills, capsules, powders, granules, sterile parenteral solutions or suspensions, or suppositories for oral, parenteral or rectal administration. For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of an imine of the present invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills or capsules. This solid preformulation composition is then subdivided into unit dosage forms of the type described above containing from 0.1 to about 500 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including polymeric acids and mixtures of polymeric acids with such materials as shellac, cetyl alcohol or cellulose acetate.

The liquid forms in which the novel compositions of the present invention may be incorporated for administration orally or by injection include aqueous solutions, suitably flavoured syrups, aqueous or oil suspensions, and flavoured emulsions with edible oils such as cottonseed oil, sesame oil, coconut oil and peanut oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, polyvinyl-pyrrolidone and gelatine.

The novel imines of this invention are useful as anticonvulsants at a dosage level of from about 0.01 to about 20 mg per kilogram of body weight, preferably about 0.05 to 2 mg/kg of body weight, on a regimen of 1 to 4 times a day.

In the treatment of neurodegeneration, a suitable dosage level is about 0.01 to 50 mg/kg/day, preferably about 0.05 to 10 mg/kg/day and especially about 0.05 to 1 mg/kg/day. The compounds may be administered on a regimen of 1 to 4 times per day.

The compounds of formula I above wherein R¹ is other than hydroxy may be prepared by a process which comprises reducing the compound of formula III: 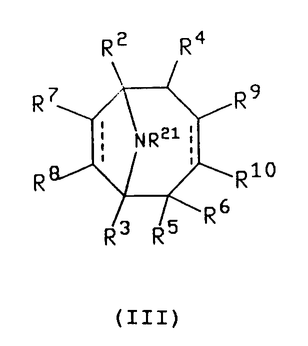
wherein R²¹ represents hydroxy or C₁₋₆ alkoxy, and R² to R¹⁰ are as defined with respect to formula I above; and, when R¹ is other than hydrogen, alkylation or alkenylation of the product. The preferred reducing agent is nascent hydrogen generated by the action of a metal, preferably zinc, with an acid, such as acetic acid. Suitable conditions for the process are a temperature of from 40°C to 100°C for 1 to about 10 hours.

The intermediate compounds of formula III may be prepared by reaction of a compound of formula IV: 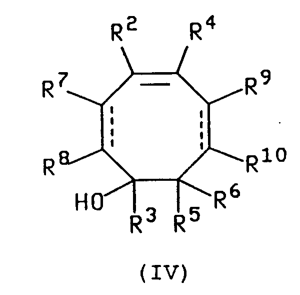
wherein R² to R¹⁰ are as defined with reference to formula I above; with a hydroxylamine derivative R²¹NH₂ in the presence of acid; followed by treatment with base such as sodium hydroxide.

In certain circumstances, the uncyclised intermediate compound of formula V will be isolated from the above process: 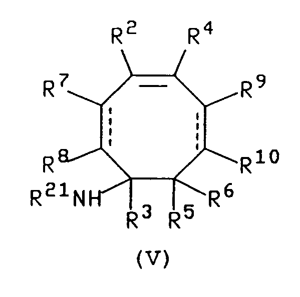
where R² to R¹⁰ and R²¹ are as hereinbefore defined. In order to effect complete conversion to the desired compound of formula III it will be necessary to treat the intermediate compound of formula V with a strong base, e.g. potassium t-butoxide.

Compounds of formula I above in which R² represents alkyl, carboxymethyl or halogen may be prepared by reacting a compound of formula VI: 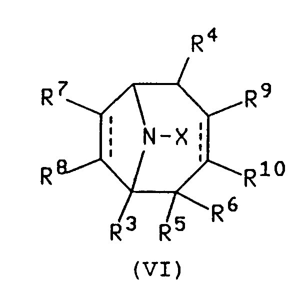
wherein R³ to R¹⁰ are as defined with respect to formula I above, and X represents a leaving group such as methoxy, p-toluenesulphonyloxy, p-toluenesulphonyl or halogen; with an anion ⁻R².

The compounds of formula I in which the bridgehead groups R² and/or R³ represent hydroxy may be prepared by methods analogous to those described in general terms in EP-A-0264183. A further method is to treat the compound of formula III above, in which R² and/or R³ is hydrogen, with manganese acetate, and subsequently to reduce the product thereby obtained with, for example, zinc in acetic acid.

This method may be illustrated by the following scheme: 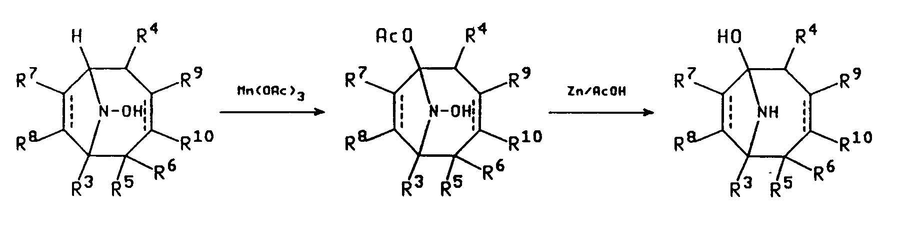
When it is desired that R¹ should be other than hydrogen, the resulting product can subsequently be alkylated or alkenylated.

The compounds of formula I in which R⁴ and/or R⁵ represent hydroxy when R⁶ represents hydrogen may be prepared by reducing the corresponding oxo or dioxo compound, in which the nitrogen atom may be protected. The reduction may be effected by treatment with, for example, diisobutylaluminium hydride (Dibal-H) in an ethereal solvent such as ether, tetrahydrofuran or 1,2-dimethoxyethane at about -90 to -60°C, preferably about -78°C, for about 1 to 3 hours.

This process produces a mixture of endo and exo hydroxy derivatives which, after deprotection, may be separated by conventional techniques such as preparative chromatography. Alternatively, in order to obtain the hydroxy compound selectively as its desired exo or endo isomer, the relevant oxo compound may be reduced with a chiral reducing agent such as D- or L-Selectride. Thus, it will be appreciated that a mixture of the exo and endo hydroxy isomers can be converted into a single desired exo or endo isomer by oxidation to the corresponding oxo compound and subsequent application of the foregoing selective reduction technique. This approach can equally be adopted for the conversion of a single exo or endo hydroxy isomer into the opposite isomer.

Further illustrative methods of preparing the exo and endo hydroxy compounds selectively in a related series are described in EP-A-0264183.

The compounds of formula I wherein R², R³, R⁴ and/or R⁵ represent fluoro may be prepared by treating the corresponding hydroxy compound with diethylaminosulphur trifluoride (DAST) in an inert organic solvent such as a chlorinated hydrocarbon, e.g. chloroform or methylene dichloride, at about 15 to 30°C for about 30 minutes to 2 hours.

In a further process of this invention, compounds having a fused saturated ring of formula VII: 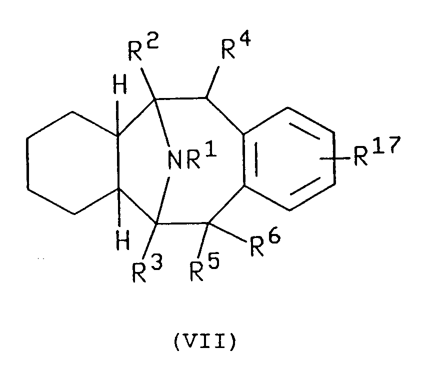
wherein R¹ to R⁶ and R¹⁷ are as hereinbefore defined; may be prepared by reducing the corresponding dibenzo compound. A preferred reducing agent is hydrogen with a platinum catalyst. Alternatively the dibenzo compound can be treated with rhodium chloride (RhCl₃.3H₂O) in an ethanolic solution at about 15 to 40°C for about 1 to 4 hours, followed by treatment with sodium borohydride (NaBH₄) at about 15 to 40°C for about 1 to 5 hours.

Compound VII can also be prepared by hydrogenating the dibenzo starting material in ethanol in the presence of a rhodium on aluminium oxide (Rh/Al₂O₃) catalyst at about 40 to 70°C starting at a pressure of about 500 to 1500 p.s.i. (3.5 x 10⁵ to 10.5 x 10⁵ kgm⁻²), for about 10 to 24 hours or until hydrogen is no longer consumed, followed by removal of catalyst and chromatographic separation of the components of the reduction product.

An additional process comprises hydrogenation of a dibenzo starting material or an N-hydroxy derivative thereof with 5% palladium-on-carbon at about 50 to 100°C and about 40 p.s.i. (195 kgm⁻²).

During any of the above reactions it may be necessary to protect any reactive groups on any of the molecules concerned with conventional protecting groups which may subsequently be removed using methods known from the art.

The compounds useful in this invention bind with a high affinity and in a reversible and saturable manner to membranes from rat brain cortex. In addition these compounds potently and selectively block responses to NMDA in a brain slice from rat cortex, and antagonise NMDA-induced seizures in the mouse.

### Binding Studies

The compounds of the invention were tested for their ability to displace a standard compound from rat brain. The standard compound employed is 5-methyl-10,11-dihydro-5H-dibenzo[a,d]cyclohepten-5,10-imine, hereinafter referred to as MK-801.

Binding of [³H]-MK-801 to rat brain in vitro was conducted in a crude synaptosomal membrane fraction (P2) prepared from rat cerebral cortex according to a modified method of Hulme et al., Molecular Pharmacology, 1978, 14, 737-750. Compounds of the invention displaced the [³H]-MK-801 binding in a concentration-dependent manner. The concentrations of the compounds of accompanying Examples 1 to 19 required to displace 50% of specific [³H]-MK-801 binding (IC₅₀) were below 5 »M in each case.

### Cortical Slice Studies

The effects of compounds of the invention on responses to NMDA were assessed using the rat cortical slice as described by Wong et al., Proc. Natl. Acad. Sci. USA, 1986, 83, 7104. The apparent equilibrium constant (K_{b}) was calculated from the righthand shift in the NMDA concentration-response curve produced by the compound under test. The compounds of accompanying Examples 1 to 8, 12, 13 and 15 to 17 were tested and their K_{b} values were found to be below 2 »M in each case.

### Antagonism of NMDLA-induced seizures

Compounds of the invention were examined for their ability to antagonise tonic seizures induced by N-methyl-DL-aspartic acid (NMDLA). Groups of 8 male Swiss-Webster mice (25 to 30 g) were injected intravenously with the test compound at various doses, 15 min before s.c. administration of NMDLA (500 mg/kg). Animals were observed for the following 30 min and the number of mice protected from tonic extension of the forelimbs noted. ED₅₀ values for the antagonism of the NMDLA induced tonic seizures were determined using probit analysis. The compounds of accompanying Examples 1, 3, 15 and 16 were tested and their ED₅₀ values were found to be below 5 mg/kg in each case.

The following Examples illustrate the preparation of compounds of this invention:

### EXAMPLE 1

### 6-Methyl-5,6,11,12-tetrahydrodibenzo[a,e]cycloocten-6,11-imine Hydrochloride

### Step A 5-Methylene-dibenzo[a,d]cycloheptene.

To a solution of dibenzosuberenone (20.6g) in diethyl ether (200ml) at 0°C under nitrogen, was added slowly with stirring, a solution of methyl lithium (100ml of a 1.4M solution in diethyl ether). The solution was kept at 0°C for 10 minutes, then water (50ml) added cautiously. The aqueous layer was discarded and the ether solution further washed with water (50ml), dried and evaporated to give crude methyl carbinol (20.2g). This was dissolved in dichloromethane (100ml), dichloroacetic acid (2ml) was added and the red solution refluxed for 3 hrs. The solution was allowed to cool, washed with saturated aqueous sodium bicarbonate solution (50ml), and water (25ml), then dried and evaporated. The crude hydrocarbon was crystallised from n-hexane (17.2g), mp 117-9°C.

### Step B 5-Hydroxymethyl-dibenzo[a,d]cycloheptene.

To a solution of 5-methylene-dibenzo[a,d]cycloheptene (15g) in dry tetrahydrofuran (50ml) at room temperature under nitrogen was added a solution of 9-BBN (160ml of a 0.5M solution in tetrahydrofuran), and the solution refluxed under nitrogen for 3h. The solution was cooled in ice, and 2N aqueous sodium hydroxide (250ml) and 30% aqueous hydrogen peroxide (50ml) were added. The cooled (ice-bath) mixture was stirred vigorously for 30 minutes, then allowed to warm to room temperature. The mixture was extracted with ether (2 x 200ml), the organic layer washed with water and brine, then dried and evaporated. Chromatography on silica gel eluting with 30% ethyl acetate in n-hexane gave pure alcohol (12.2g), mp 86-7°C.

### Step C 5-p-Toluenesulphonyloxymethyl-dibenzo[a,d]cycloheptene

To a solution of 5-hydroxymethyl-dibenzo[a,d]cycloheptene (11.1g) in dichloromethane (100ml) was added p-toluenesulphonylchloride (9.5g) and pyridine (7.9g, dried over KOH), and the solution stirred with drying tube protection. A slight exothermic reaction was evident. After 2h the reaction mixture was washed with water (50ml) and the organic layer dried and evaporated. The residue was triturated with warm hexane and the solid product collected and washed with hexane, then dried in vacuo to give the required tosylate (17.1g), mp 139-40°C.

### Step D 5-Hydroxy-dibenzo[a,e]cyclooctene

To a solution of 5-p-toluenesulphonyloxymethyl-dibenzo[a,d] cycloheptene (17g) in glacial acetic acid (150ml) was added anhydrous sodium acetate (8.2g) and the solution refluxed with stirring for 4h. The solvents were removed in vacuo, the residue dissolved in methanol (100ml), cooled to 0°C, and enough solid potassium hydroxide added to give pH 14. The suspension was stirred at room temperature for 2h, then the methanol was removed in vacuo and the residue partitioned between ether (200ml) and water (50ml). The organic layer was washed three times with water (50ml), then dried and evaporated to give the crude alcohol which was crystallised from n-hexane/5% ethyl acetate (10.2g), mp 120-1°C.

### Step E 5-Oxo-dibenzo[a,e]cyclooctene

To a solution of oxalyl chloride (5ml) in dry dichloromethane (75ml) at -78°C under nitrogen was added, dropwise, dimethyl sulphoxide (8.75ml), and the solution stirred at -78°C for 15 minutes. A solution of 5-hydroxy-dibenzo[a,e]cyclooctene (11.1g) in dichloromethane (75ml) was added over 5 minutes at -78°C and the solution stirred for 15 minutes, then triethylamine (30ml) was added and the mixture allowed to warm to room temperature. The mixture was washed with water (100ml), 1N hydrochloric acid (50ml), water (100ml), brine (50ml) then dried and evaporated to give the crude ketone which was recrystallised from ethyl alcohol/hexane (10.0g), mp 112-6°C.

### Step F 5-Hydroxy-5-methyl-dibenzo[a,e]cyclooctene

To a stirred solution of 5-oxo-dibenzo[a,e]cyclooctene (10.0g) in dry diethyl ether at 0°C, under nitrogen, was added over 5 minutes, through a canula, a solution of methyl lithium (40ml of a 1.4M solution in diethyl ether). After a further 10 minutes stirring water (40ml) was added cautiously and the separated aqueous layer discarded. The organic layer was washed with water, then dried and evaporated. Crystallisation from diethyl ether/hexane gave the pure alcohol (6.2g), mp 108-109°C.

### Step G 13-Hydroxy-5-methyl-5,6,11,12 tetrahydrodibenzo[a,e]cycloocten-6-11-imine

To a suspension of hydroxylamine hydrochloride (2.64g) and anhydrous sodium acetate (3.11g) in dichloromethane (25ml) was added 5-hydroxy-5-methyldibenzo[a,e]cyclooctene (0.90g). The mixture was stirred vigorously and brought to reflux under nitrogen. To the vigorously stirred refluxing suspension was added, over 10 minutes, dichloroacetic acid (8ml) in dichloromethane (50ml). The mixture was refluxed for 2h, then allowed to cool to room temperature and 2N sodium hydroxide (60ml) added. The organic layer was washed with water and brine, then dried and evaporated. Crystallisation from ethyl acetate/hexane gave the desired imine (370mg), mp 159-63°C.

### Step H 6-Methyl-5,6,11,12-tetrahydrodibenzo[a,e]cycloocten-6,11-imine

To a solution of the hydroxy imine (Step G, 200mg) in glacial acetic acid (3ml) was added zinc powder (500mg) and the suspension stirred at 68°C overnight. The mixture was filtered, the cake washed with acetic acid (1ml), then the filtrate was evaporated and partitioned between ether (10ml) and 2N aqueous sodium hydroxide (10ml). The ether layer was washed with water and brine, then dried and evaporated to give the crude product as a buff solid. This was recrystallised from warm n-hexane to give 6-methyl-5,6,11,12-tetrahydrodibenzo[a,e]cycloocten-6,11-imine (110mg), mp 125-6°. The structure was confirmed by single crystal X-ray diffraction.

### Step I 6-Methyl-5,6,11,12-tetrahydrodibenzo[a,e]cycloocten-6,11-imine hydrochloride

To a solution of the imine (Step H, 50mg) in ethyl acetate (2ml) at room temperature was added a solution of saturated hydrogen chloride in ethyl acetate (2ml). After 2 minutes a white precipitate was deposited. This was collected by filtration, washed successively with ethyl acetate and dry ether and dried in vacuo to give pure 6-methyl-5,6,11,12-tetrahydrodibenzo[a,e]cycloocten-6,11-imine hydrochloride (38mg, mp 270-275°C), δ (CDCl₃, 360MH_{z}) 2.03 (3H, s, CH₃), 2.92 (1H, d, J = 16.6Hz, C(CH₃)-CH_{A}H_{B}), 3.17 (1H, dd, J = 16.8 and 5.7Hz, CH_{C}H_{D}-CH_{E}(NH), 4.10 (1H, d, J = 16.8Hz, CH_{C}H_{D}-CH_{E}(NH)), 4.24 (1H, d, J = 16.6Hz C(CH₃)-CH_{A}H_{B}), 5.13 (1H, m, CH_{C}H_{D}-CH_{E}(NH)), 6.79-7.18 (8H, m, ArH).

### EXAMPLE 2

### 9-Bromo-6-methyl-5,6,11,12-tetrahydrodibenzo[a,e]cycloocten-6,11-imine hydrochloride

### Step A 2-Bromo-5-methylene-dibenzo[a,d]cycloheptene

A solution of 2-bromo-dibenzo[a,d]cyclohepten-5-one (7.7g) in anhydrous tetrahydrofuran (200ml) was cooled to 0°C and methyllithium (19.5ml of a 1.4 molar solution) was added by syringe. After 15 minutes, water (50ml) was added dropwise and the reaction mixture extracted with diethyl ether (3 x 100ml). The combined organic phases were dried over sodium sulphate, filtered and concentrated in vacuo to give a colourless oil (6.5g). δ (360MH_{z}, CDCl₃) 1.6 (3H, br, s, CH₃), 6.9 (1H, d, J = 12Hz CH_{A}=CH_{B}), 7.0 (1H, d, J = 12Hz, CH_{A}=CH_{B}), 7.4-8.0 (7H, m, ArH). m/e 302 and 300 (M⁺). This was dissolved in dichloromethane (50ml) with dichloroacetic acid (2.5ml) and refluxed for 9h. The solvent was removed in vacuo and the residue chromatographed on SiO₂ with 5% ethyl acetate in hexane as eluent to give the title compound as a colourless solid (5.8g, mp 112-114°C). δ (360MHz, CDCl₃) 5.24 (1H, d, J = 1.4Hz, = CH_{A}H_{B}), 5.26 (1H, d, J = 1.4Hz =CH_{A}H_{B}), 6.71 (1H, d, J = 12Hz, CH_{C}=CH_{D}), 6.85 (1H, d, J = 12Hz, CH_{C}=CH_{D}), 7.2-7.5 (7H, m, ArH). m/e 284 and 282 (M⁺).

### Step B 2-Bromo-5-hydroxymethyl-dibenzo[a,d]cycloheptene

To a solution of 2-bromo-5- methylene-dibenzo[a,d]cycloheptene (5.7g) in dry THF (20ml) under an inert atmosphere, was added 9-borabicyclo[3,3,1]nonane (4.4ml of a 0.5 molar solution in THF) at ambient temperature, and the reaction mixture was then refluxed for 3h. After cooling in an ice bath the reaction was quenched by the slow addition of 2N sodium hydroxide solution (100ml) and 30% hydrogen peroxide (20ml). After stirring vigorously at 0°C for 45 minutes the reaction mixture was allowed to warm to room temperature over a 2h period then extracted with diethyl ether (3 x 200ml). The combined organic layers were washed with brine (1 x 100ml) dried (Na₂SO₄), filtered and concentrated in vacuo to leave a residue which was purified by chromatography on flash silica with 30% ethyl acetate in hexane as eluent to give the title compound (6.0g) as a colourless oil. δ (360MHz, CDCl₃) 3.81 (2H, d, J = 7.9Hz, CH-CH₂OH), 4.17 (1H, t, J = 7.9Hz, CH-CH₂OH), 6.75 (1H, d, J = 12Hz, CH_{A}=CH_{B}), 6.89 (1H, d, J = 12Hz, CH_{A}=CH_{B}), 7.15-7.48 (7H, m, ArH).

### Step C 2-Bromo-5-p-toluenesulphonyloxymethyl-dibenzo[a,d]cycloheptene

To a solution of 2-bromo-5-hydroxymethyl-dibenzo[a,d]cycloheptene (6.1g) in dry dichloromethane (60ml) was added p-toluenesulphonyl chloride (4.9g), pyridine (4g) and 4-dimethylamino pyridine (100mg). The reaction mixture was refluxed for 14h, cooled, washed with water (2 x 50ml) dried (Na₂SO₄), filtered and concentrated in vacuo. The residue. was purified by flash silica chromatography using 10% ethyl acetate in hexane as eluent to give the title compound as a colourless oil (8.2g). δ (360MHz, CDCl₃) 2.46 (3H, s, CH₃), 4.12 (1H, dd J = 6.2 and 3.5Hz, CH-CH₂-O), 4.24-4.32 (2H, m, CH-CH₂-O), 6.50 (1H, d, J = 11.9Hz, CH_{A}=CH_{B}), 6.73 (1H, d, J = 11.9Hz, CH_{A}=CH_{B}), 7.10-7.40 (11H, m, ArH).

### Step D 2-Bromo-5-hydroxy-dibenzo[a,e]cyclooctene and 2-bromo-6-hydroxy dibenzo[a,e]cyclooctene

To a solution of 2-bromo-5-p-toluenesulphonyloxymethyl-dibenzo[a,d]cycloheptene (8.1g) in glacial acetic acid (75ml) was added anhydrous sodium acetate (2.9g). The reaction mixture was refluxed for 24h then the solvent removed under vacuum to leave a residue which was partitioned between dichloromethane (3 x 100ml) and water (100ml). The combined organic layers were dried (Na₂SO₄), filtered and concentrated in vacuo to leave a residue which was dissolved in 75% aqueous methanol (100ml) and treated with potassium hydroxide (4g). After 14h the reaction mixture was concentrated in vacuo and the residue partitioned between water (50ml) and diethyl ether (3 x 50ml). The combined organic layers were dried (Na₂SO₄), filtered and concentrated in vacuo. Chromatography of the residue on flash silica with 10% ethyl acetate in hexane as eluent gave the less polar isomer, 2-bromo-5-hydroxy-dibenzo[a,e]cyclooctene (2.54g, m.p. 148-151°C). δ (360MHz, CDCl₃) 3.26 (1H, dd, J = 13.9 and 9.9Hz, CH_{A}H_{B}-CHOH), 3.46 (1H, dd, J = 13.9 and 6.2 Hz, CH_{A}H_{B}-CHOH), 5.24 (1H, dd, J = 9.9 and 6.2Hz, CHOH), 6.76 (1H, d, J = 12.1Hz, CH_{C}=CH_{D}), 6.87 (1H, d, J = 12.1Hz, CH_{C}=CH_{D}), 7.09-7.35 (7H, m, ArH). m/e 302 and 300 (M⁺); and as an oil, the more polar isomer 2-bromo-6-hydroxy-dibenzo[a,e]cyclooctene (1.04g). δ (360MHz, CDCl₃) 3.25 (1H, dd, J = 13.8 and 10.0Hz, CH_{A}H_{B}-CHOH), 3.40 (1H, dd, J = 13.8 and 6.4Hz, CH_{A}H_{B}-CHOH), 5.26 (1H, dd, J = 10.0 and 6.4Hz, CH_{A}H_{B}-CHOH), 6.75 (1H, d, J = 12.1Hz, CH_{C}=CH_{D}), 6.90 (1H, d, J = 12.1Hz, CH_{C}=CH_{D}), 7.08-7.45 (7H, m, ArH). m/e 302 and 300 (M⁺).

### Step E 2-Bromo-5-oxo-dibenzo[a,e]cyclooctene

To a solution of 2-bromo-5-hydroxy-dibenzo[a,e]cyclooctene (2.3g) in dry dichloromethane (100ml) was added pyridinium dichromate (5.75g) and crushed 4A molecular sieves (3g). After stirring at room temperature for 14h, diethyl ether (200ml) was added and the reaction mixture was filtered through a plug of celite. The solvent was removed in vacuo and the residue purified by chromotagraphy on flash silica with 5% ethyl acetate in hexane as eluent to give as a colourless solid, the title compound (2.1g, m.p. 121-123°C). δ (360MHz, CDCl₃) 4.03 (2H, s, CH₂), 6.92 (1H, d, J = 12.8 Hz, CH_{A}=CH_{B}), 7.09 (1H, d, J = 12.8Hz, CH_{A}=CH_{B}), 7.20-7.56 (6H, m, ArH), 8.08 (1H, d, J = 8.5Hz, BrC=CH-CH=C-C=O). m/e 300 and 298 (M⁺).

### Step F 9-Bromo-13-hydroxy-6-methyl-5,6,11,12-tetrahydrodibenzo[a,e]cycloocten-6,11-imine

To a solution of 2-bromo-5-oxo-dibenzo[a,e]cyclooctene (1g) in dry diethyl ether (20ml) at 0°C under an atmosphere of nitrogen was added methyl lithium (2.2ml of a 1.6 molar solution). After 3h at room temperature the reaction mixture was cooled to 0°C, quenched dropwise with water (20ml) and extracted with diethyl ether (3 x 30ml). The combined organic layers were dried (Na₂SO₄), filtered and evaporated in vacuo to leave an oil (1.03g), which was dissolved in dry dichloromethane (15ml) and added to a solution containing dichloroacetic acid (4.1ml), sodium acetate (2.7g) and hydroxylamine hydrochloride (2.3g) in dichloromethane (55ml) which had been prepared 2 hours previously. The reaction mixture was stirred at room temperature for 6h then refluxed for a further 6h. After cooling, 2N potassium hydroxide solution was added until the pH was 14 and the two-phase mixture was vigorously stirred for 30 minutes. The organic layer was separated, washed with water (1 x 50ml) and brine (1 x 50ml), dried (Na₂SO₄), filtered and concentrated in vacuo. The crude residue was triturated with hot hexane to produce the required product as a white crystalline solid which was collected by filtration (0.17g, m.p. 147-149°C). The spectrum (360MHz, CDCl₃) showed a complex mixture containing two atropisomers.

### Step G 9-Bromo-6-methyl-5,6,11,12-tetrahydrodibenzo[a,e]cycloocten-6,11-imine hydrochloride

To a solution of 9-bromo-13-hydroxy-6-methyl-5,6,11,12-tetrahydrodibenzo[a,e]cycloocten-6,11-imine (0.1g) in glacial acetic acid (4ml) was added zinc dust (0.2g). The reaction mixture was heated at 70°C under an inert atmosphere for 6h then filtered and concentrated in vacuo to leave a residue which was partitioned between dichloromethane (30ml) and dilute sodium hydroxide solution (30ml). The organic layer was separated, dried (Na₂SO₄), filtered and evaporated to leave a residue which was dissolved in 5 molar hydrogen chloride in ethyl acetate (15ml). The solvent was evaporated to leave a solid which was recrystallised from ethyl acetate/diethylether. The title compound (0.07g, m.p. 172-175°C) was collected by filtration and dried under high vacuum. δ (DMSO, 360MHz) 1.87 (3H, s, CH₃), 3.11 (1H, d, J = 16.4Hz, PhCH_{A}H_{B}-C(NH)CH₃), 3.25 (1H, dd, J = 16.7 and 5.0Hz, PhCH_{C}H_{D}-C(NH)H_{E}), 3.69 (1H, d, J = 16.7Hz, PhCH_{C}H_{D}-C(NH)H_{E}), 3.84 (1H, d, J = 16.4Hz, PhCH_{A}H_{B}-C(NH)CH₃), 5.16 (1H, d, J = 5.0Hz, PhCH_{C}H_{D}-C(NH)H_{E}), 6.88-6.98 (4H, m, ArH), 7.19 (1H, d, J = 8.1Hz, CH_{F}=CH_{G}-CBr=CH_{H}), 7.37 (1H, dd, J = 8.1 and 1.8Hz, CH_{F}=CH_{G}-CBr-CH_{H}), 7.51 (1H, d, J = 1.8Hz, CH_{F}=CH_{G}-CBr=CH_{H}). n.O.e's observed from CH₃ to H_{F} and H_{H} to H_{E}.

### EXAMPLE 3

### 2-Bromo-6-methyl-5,6,11,12-tetrahydrodibenzo[a,e]cycloocten-6,11-imine hydrochloride

### Step A 2-Bromo-6-oxo-dibenzo[a,e]cyclooctene

To a solution of 2-bromo-6-hydroxy-dibenzo[a,e]cyclooctene (1.0g) in dry dichloromethane (50ml) was added pyridinium dichromate (2.75g) and crushed 4A molecular sieves (1.5g). After stirring at room temperature for 14h, diethyl ether (150ml) was added and the reaction mixture was filtered through a plug of celite. The solvent was removed in vacuo and the residue purified by chromatography on flash silica with 5% ethyl acetate in hexane as eluent to give, as a colourless solid, the title compound (0.76g, m.p. 147-150°C). δ (360MHz, CDCl₃) 4.01 (2H, s, CH₂-C=O), 6.96 (1H, d, J = 12.7Hz, CH_{A}=CH_{B}), 7.06 (1H, d, J = 12.7Hz, CH_{A}=CH_{B}), 7.25-7.52 (6H, m, ArH), 8.21 (1H, d, J = 8.0Hz, C(O)C=CH-CH=CH-CH). m/e 302 and 300 (M⁺).

### Step B 2-Bromo-13-hydroxy-6-methyl-5,6,11,12-tetrahydrodibenzo[a,e]cycloocten-6,11-imine

To a solution of 2-bromo-6-oxo-dibenzo[a,e]cyclooctene (0.7g) in dry diethyl ether (30ml) at 0°C under atmosphere of nitrogen was added methyl magnesium bromide (2ml of a 3 molar solution). After 4h at room temperature the reaction mixture was cooled to 0°C, quenched by the dropwise addition of saturated ammonium suphate solution (20ml) and extracted into diethyl ether (3 x 30ml). The combined organic layers were dried (Na₂SO₄), filtered and evaporated to leave an oil (0.72g) which was dissolved in dry dichloromethane (10ml) and added to a solution containing dichloroacetic acid (3.1ml), sodium acetate (2.1g) and hydroxylamine hydrochloride (1.7g) in dichloromethane (40ml) which had been prepared 2 hours previously. The reaction mixture was stirred at room temperature for 6h then refluxed for a further 6h. After cooling, 2N potassium hydroxide solution was added until the pH was 14 and the two phase mixture was stirred vigorously for 30 minutes. The organic layer was separated, washed with water (1 x 40ml) and brine (1 x 40ml), dried (Na₂SO₄), filtered and concentrated in vacuo. The crude residue was purified by chromatogrpahy on flash silica with 30% ethyl acetate in hexane to give, as a colourless foam, the title compound (0.15g). The nmr spectrum (360MHz, CDCl₃) showed a mixture of two atropisomers. m/e 331 and 329 (M⁺).

### Step C 2-Bromo-6-methyl-5,6,11,12-tetrahydrodibenzo[a,e]cycloocten-6,11-imine hydrochloride

To a solution of 2-bromo-13-hydroxy-6-methyl-5,6, 11,12-tetrahydrodibenzo[a,e]cycloocten-6,11-imine (0.14g) in glacial acetic acid (5ml) was added zinc dust (0.28g). The reaction mixture was heated at 65°C under an atmosphere of nitrogen for 6h then filtered and concentrated in vacuo to leave a residue which was partitioned between dichloromethane (30ml) and dilute sodium hydroxide solution (30ml). The organic layer was separated, dried (Na₂SO₄), filtered and evaporated to leave a residue which was dissolved in 5 molar hyrogen chloride in ethyl acetate (20ml). The solvent was evaporated to leave a solid which was recrystallised from ethyl acetate/diethyl ether. The title compound (0.04g, m.p. 189-191°C) was collected by filtration and dried under high vacuum. δ (DMSO, 360MHz) 1,88 (3H, s, CH₃), 3.12 (1H, d, J = 16.5Hz, C(CH₃)CH_{A}H_{B}), 3.36 (1H, dd, J = 16.7 and 5.9 Hz, CH_{C}H_{D}CH_{E}(NH)), 3.72 (1H, d, J = 16.7Hz, CH_{C}H_{D}CH_{E}(NH), 3,83 (1H, d, J = 16.5Hz, C(CH₃)-CH_{A}H_{B}), 5.19 (1H, d, J = 5.9Hz, CH_{C}H_{D}CH_{E}(NH), 6.84 (1H, d, J = 8.1.Hz, CH_{F}=CH_{G}-CBr=CH), 7.12 (1H, dd, J = 8.1 and 2.0Hz, CH_{F}=CH_{G}-CBr=CH), 7.17-7.42 (5H, m, ArH). n.O.e.'s observed from CH₃ and from H_{E} to the aromatic protons on the unsubstituted ring; and from H_{F} to H_{A} and H_{F} to H_{G}.

### EXAMPLE 4

### 9-Chloro-6-methyl-5,6,11,12-tetrahydrodibenzo[a,e] cycloocten-6,11-imine hydrochloride

### Step A 2-Chloro-5-methylene-dibenzo[a,d]cycloheptene

A solution of 2-chloro-dibenzo[a,d]cyclohepten-5-one (8.7g) in anhydrous diethyl ether (200ml) was cooled to 0°C and methyllithium (38ml of a 1.4 molar solution) was added by syringe. After 20 minutes, water (50ml) was added dropwise and the reaction mixture extracted with diethyl ether (3 x 100ml). The combined organic layers were dried over sodium sulphate, filtered and concentrated in vacuo to give a colourless oil (9.3g). This was dissolved in dichloromethane (200ml) with dichloroacetic acid (3ml) and refluxed for 1h. The mixture was cooled, washed with saturated sodium hydrogen carbonate solution (1 x 100ml) and brine (1 x 100ml), dried (Na₂SO₄), filtered and concentrated in vacuo to give the title compound as a colourless solid (8.4g, m.p. 100-102°C) δ (360MHz, CDCl₃) 5.24 (1H, d, J = 1.4Hz, CH_{A}H_{B}), 5.26 (1H, d, J = 1.4Hz, CH_{A}H_{B}), 6.72 (1H, d, J = 12Hz, CH_{C}=CH_{D}), 6.86 (1H, d, J = 12Hz, CH_{C}=CH_{D}), 7.24-7.39 (7H, m, ArH). m/e 240 and 238 (M⁺).

### Step B 2-Chloro-5-hydroxymethyl-dibenzo[a,d] cycloheptene

To a solution of 2-chloro-5-methylene-dibenzo[a,d] cycloheptene (8.3g) in dry tetrahydrofuran (30ml), under an inert atmosphere, was added 9-borabicyclo [3,3,1] nonane (71ml of a 0.5 molar solution in tetrahydrofuran) at ambient temperature and the reaction mixture was then refluxed for 3h. After cooling, the reaction was quenched by the slow addition of 2N sodium hydroxide solution (180ml) and 30% hydrogen peroxide (36ml). After stirring vigorously at 0°C for 45 minutes the reaction mixture was allowed to warm to room temperature over a 2h period then extracted into diethyl ether (3 x 250ml). The combined organic layers were washed with brine (1 x 150ml), dried (Na₂SO₄), filtered and concentrated in vacuo to leave a residue which was purified by chromatography on flash silica with 40% ethyl acetate in hexane as eluant to give the title compound (8.1g) as a colourless oil. δ (360MHz, CDCl₃) 3.80 (2H, d, J = 7.9Hz CH-CH₂OH), 4.18 (1H, t, J = 7.9Hz, CH-CH₂OH), 6.76 (1H, d, J = 11.9Hz, CH_{A}=CH_{B}) 6.89 (1H, d, J = 11.9Hz, CH_{A}=CH_{B}), 7.24-7.38 (7H, m, ArH); m/e 258 and 256 (M⁺).

### Step C 2-Chloro-5-hydroxy-dibenzo[a,e]cyclooctene and 2-chloro-6-hydroxy-dibenzo[a,e]cyclooctene

To a solution of 2-chloro-5-hydroxymethyl-dibenzo [a,d]cycloheptene (8.1g) in dry dichloromethane (70ml) was added p-toluene sulphonyl chloride (6.7g), pyridine (2.9ml) and 4-dimethylamino pyridine (100mg). The reaction mixture was refluxed for 14h, cooled, washed with water (2 x 50ml), dried (Na₂SO₄), filtered and concentrated in vacuo to leave an oil which was purified by chromatography on flash silica with 10% ethyl acetate in hexane as eluent. The purified tosylate was dissolved in glacial acetic acid (70ml) with anhydrous sodium acetate (7.4g) and refluxed for 14h. The mixture was concentrated in vacuo to leave a residue which was partitioned between dichloromethane (3 x 100ml) and water (100ml). The combined organic layers were dried (Na₂SO₄), filtered and concentrated in vacuo to leave a residue which was dissolved in 75% aqueous methanol (150ml) and treated with potassium hydroxide (10g). After 14h the reaction mixture was concentrated in vacuo and the residue partitioned between water (75ml) and diethyl ether (3 x 75ml). The combined organic layers were dried (Na₂SO₄), filtered and concentrated in vacuo. Chromatography of the residue on flash silica with 15% ethyl acetate in hexane as eluent gave the less polar isomer 2-chloro-5-hydroxydibenzo[a,e]cyclooctene (2.9g, m.p. 148-149°C). δ (360MHz, CDCl₃) 3.26 (1H, dd, J = 14.0 and 9.9Hz, CH_{A}H_{B}-CHOH), 3.46 (1H, dd, J = 14.0 and 6.3Hz, CH_{A}H_{B}-CHOH), 5.27 (1H, dd, J = 9.9 and 6.3Hz, CH_{A}H_{B}-CHOH), 6.77 (1H, d, J = 12.0Hz, CH_{C}=CH_{D}), 6.87 (1H, d, J = 12.0Hz, CH_{C}=CH_{D}), 7.08-7.41 (7H, m, ArH), m/e 258 and 256 (M⁺); and, as an oil, the more polar isomer 2-chloro-6-hydroxy-dibenzo[a,e]cyclooctene (1.7g) δ (360MHz, CDCl₃) 3.27 (1H, dd, J = 13.8 and 10.0Hz, CH_{A}H_{B}-CHOH), 3.41 (1H, dd, J = 13.8 and 6.3Hz, CH_{A}H_{B}-CHOH), 5.25 (1H, dd, J = 10.0 and 6.3Hz, CH_{A}H_{B}-CHOH), 6.75 (1H, d, J = 12.0Hz, CH_{C}=CH_{D}), 6.90 (1H, d, J = 12.0Hz, CH_{C}=CH_{D}), 7.07-7.44 (7H, m, ArH). m/e 258 and 256 (M⁺).

### Step D 2-Chloro-5-oxo-dibenzo[a,e]cyclooctene

To a solution of 2-chloro-5-hydoxy-dibenzo[a,e] cyclooctene (1.5g) in dry dichloromethane (60ml) was added pyridinium dichromate (5g) and crushed 4A molecular sieves (2.5g). After stirring at room temperature for 14h, diethyl ether (150ml) was added and the reaction mixture was filtered through a plug of celite. The solvent was removed in vacuo and the residue purified by chromatography on flash silica with 5% ethyl acetate in hexane as eluent to give, as a colourless oil, the title compound (1.4g). δ (360MHz, CDCl₃) 4.04 (2H, s, CH₂), 6.93 (1H, d, J = 12.9Hz, CH_{A}=CH_{B}), 7.10 (1H, d, J = 12.9Hz, CH_{A}=CH_{B}), 7.22-7.42 (6H, m, ArH), 8.17 (1H, d, J = 8.4Hz, ClC=CH-CH=C-C=O). m/e 256 and 254 (M⁺).

### Step E 9-Chloro-13-hydroxy-6-methyl-5,6,11,12-tetrahydrodibenzo[a,e]cycloocten-6,11-imine

To a solution of 2-chloro-5-oxo-dibenzo[a,e]cyclooctene (1.4g) in dry diethyl ether (50ml) at 0°C under an atmosphere of nitrogen was added methyllithium (4ml of a 1.6 molar solution). After 3h at room temperature the reaction mixture was cooled to 0°C, quenched dropwise with water (30ml) and extracted with diethyl ether (3 x 30ml). The combined organic layers were dried (Na₂SO₄), filtered and evaporated to leave an oil (1.73g), which was dissolved in dry dichloromethane (15ml) and added to a solution containing dichloracetic acid (12.2ml) sodium acetate (5.2g) and hydroxylamine hydrochloride (4.4g) in dichloromethane (70ml) which had been prepared 2 hours previously. The reaction mixture was stirred at room temperature for 2h then refluxed for a further 3h. After cooling, 2N potassium hydroxide solution was added until the pH was 14 and the two phase mixture was stirred vigorously for 30 minutes. The organic layer was separated, washed with water (1 x 60ml) and brine (1 x 60ml), dried (Na₂SO₄), filtered and concentrated in vacuo. The crude residue was triturated with hot hexane to produce the title compound (0.2g, m.p. 182°C). The nmr spectrum (360MHz, CDCl₃) showed a mixture of two atropisomers.

### Step F 9-Chloro-6-methyl-5,6,11,12-tetrahydrodibenzo [a,e]cycloocten-6,11-imine hydrochloride

To a solution of 9-chloro-13-hydroxy-6-methyl-5,6,11,12-tetrahydrodibenzo[a,e]cycloocten-6,11-imine (0.1g) in glacial acetic acid (3ml) was added zinc dust (0.2g). The reaction mixture was heated at 70°C under an inert atmosphere for 11h then filtered and concentrated in vacuo to leave a residue which was partitioned between dichloromethane (30ml) and dilute sodium hydroxide solution (30ml). The organic layer was separated, dried (Na₂SO₄), filtered and evaporated to leave an oil which was dissolved in 5 molar hydrogen chloride in ethyl acetate (15ml). The solvent was evaporated to leave a solid which was recrystallised from ethyl acetate/diethyl ether. The title compound (0.064g, m.p. 187-191°C) was collected by filtration and dried under high vacuum. δ (DMSO, 360MHz) 1.89 (3H, s, CH₃), 3.09 (1H, d, J = 16.5Hz, PhCH_{A}H_{B}-C(NH)CH₃), 3.27 (1H, dd, J = 16.7Hz and 4.91Hz, PhCH_{C}H_{D}-C(NH)CH₃), 3.73 (1H, d, J = 16.7Hz, CH_{C}H_{D}-C(NH)H_{E}), 3.89 (1H, d, J = 16.5Hz, CH_{A}H_{B}-C(NH)CH₃), 5.17 (1H, d, J = 4.9Hz, PhCH_{C}H_{D}-C(NH)H_{E}), 6.90-6.97 (4H, m, ArH), 7.23-7.27 (2H, m, ArH), 7.37 (1H, s, CH_{F}=CBr-CH=CH). n.o.e.'s observed from H_{E} to H_{F} and CH₃ to δ 7.25 (one of the two other protons on the chlorine substituted aromatic ring). m/e 271 and 269 (M⁺).

### EXAMPLE 5

### 2-Chloro-6-methyl-5,6,11,12-tetrahydrodibenzo[a,e] cycloocten-6,11-imine hydrochloride

### Step A 2-Chloro-6-oxo-dibenzo[a,e]cyclooctene

To a solution of 2-chloro-6-hydroxy-dibenzo[a,e] cyclooctene (1.6g) in dry dichloromethane (80ml) was added pyridinium dichromate (4.3g) and crushed 4A molecular sieves (2.5g). After stirring at room temperature for 14h, diethyl ether (200ml) was added and the reaction mixture was filtered through a plug of celite. The solvent was removed in vacuo and the residue purified by chromatography on flash silica with 5% ethyl acetate in hexane as eluent to give, as a colourless oil, the title compound (0.7g). δ (360MHz, CDCl₃) 4.03 (2H, s, CH₂-C=O), 6.97 (1H, d, J = 12.8Hz, CH_{A}=CH_{B}), 7.06 (1H, d, J = 12.8Hz, CH_{A}=CH_{B}), 7.23-7.53 (6H, m, aromatics), 8.22 (1H, d, J = 7.9Hz, C(O)C=CH-CH=CH-CH). m/e 256 and 254 (M⁺).

### Step B 2-Chloro-13-hydroxy-6-methyl-5,6,11,12-tetrahydrodibenzo[a,e]cycloocten-6,11-imine

To a solution of 2-bromo-6-oxo-dibenzo[a,e] cyclooctene (0.67g) in dry diethyl ether (40ml) at 0°C under nitrogen was added methyl magnesium bromide (1.8ml of a 3 molar solution). After 4h at room temperature the reaction mixture was cooled to 0°C, quenched by the dropwise addition of saturated ammonium sulphate solution (15ml) and extracted into diethyl ether (3 x 25ml). The combined organic layers were dried (Na₂SO₄), filtered and evaporated to leave an oil which was dissolved in dry dichloromethane (10ml) and added to a solution containing dichloroacetic acid (3.2ml), sodium acetate (2.1g) and hydroxylamine hydrochloride (1.8g) in dichloromethane (35ml), which had been prepared 2h previously. The reaction mixture was stirred at room temperature for 6h then refluxed for a further 8h. After cooling, 2N potassium hydroxide was added until the pH was 14 and the two phase mixture was stirred vigorously for 30 minutes. The organic layer was separated, washed with water (1 x 40ml) and brine (1 x 40ml), dried (Na₂SO₄), filtered and concentrated in vacuo. The crude residue was purified by chromatography on flash silica with 30% ethyl acetate in hexane to give, as a colourless foam, the title compound (0.08g). The nmr spectrum (360MHz, CDCl₃) showed a mixture of two atropisomers. m/e 287 and 285 (M⁺).

### Step C 2-Chloro-6-methyl-5,6,11,12-tetrahydrodibenzo[a,e]cycloocten-6,11-imine hydrochloride

To a solution of 2-chloro-13-hydroxy-6-methyl-5,6,11,12-tetrahydrodibenzo[a,e]cycloocten-6,11-imine (0.065g) in glacial acetic acid (5ml) was added zinc dust (0.13g). The reaction mixture was heated at 65°C under an atmosphere of nitrogen for 8h, then filtered and concentrated in vacuo to leave a residue which was partitioned between dichloromethane (30ml) and dilute sodium hydroxide solution (30ml). The organic layer was separated, dried (Na₂SO₄), filtered and concentrated in vacuo to leave a residue which was dissolved in 5 molar hydrogen chloride in ethyl acetate (15ml). The solvent was evaporated to leave a solid which was recrystallised from ethyl acetate/diethyl ether. The title compound (0.043g, m.p. 174-176°C) was collected by filtration and dried under high vacuum. δ (DMSO, 360MHz) 1.89 (3H, s, CH₃), 3.14 (1H, d, J = 16.5Hz, C(CH₃)CH_{A}H_{B}), 3.34 (1H, dd, J = 16.8 and 6.1Hz, CH_{C}H_{D}CH_{E}(NH)), 3.70 (1H, d, J = 16.8Hz, CH_{C}H_{D}CH_{E}(NH)), 3.84 (1H, d, J = 16.5Hz, C(CH₃)CH_{A}H_{B}), 5.20 (1H, d, J = 6.1Hz, CH_{C}H_{D}CH_{E}(NH)), 6.90 (1H, d, J = 8.2Hz, CH_{F}=CH_{G}-CCl=CH_{H}), 6.99 (1H, dd, J = 8.1 and 2.1Hz, CH_{F}=CH_{G}-CCl=CH_{H}), 7.04 (1H, d, J = 2.1 Hz, CH_{F}=CH_{G}-CCl=CH_{H}), 7.17-7.27 (4H, m, ArH). n.O.e.'s observed from CH₃ and from H_{E} to the aromatic protons on the unsubstituted ring.

### EXAMPLE 6

### 8-Bromo-6-methyl-5,6,11,12-tetrahydrodibenzo[a,e] cycloocten-6,11-imine hydrochloide

### Step A 3-Bromo-5-methylene-dibenzo[a,d]cycloheptene

To a solution of 3-bromo-dibenzo[a,d]cyclohepten-5-one (4.1g) in anhydrous tetrahydrofuran (50ml) at 0°C under nitrogen was added methyllithium (10.7ml of a 1.4 molar solution) by syringe. After 15 minutes, water (30ml) was added dropwise and the reaction mixture extracted with diethyl ether (3 x 100ml). The combined organic layers were dried (Na₂SO₄), filtered and concentrated in vacuo to give a colourless oil (4.2g) which was dissolved in dichloromethane (30ml) with dichloroacetic acid (1ml) and refluxed for 9h. The solvent was removed under vacuum to leave a residue which was purified by chromatography on flash silica with 5% ethyl acetate in hexane as eluent to give, as a colourless oil, the title compound (3.9g). δ (360MHz, CDCl₃) 5.28 (2H, s, C=CH₂), 6.75 (1H, d, J = 12Hz, CH_{A}=CH_{B}), 6.84 (1H, d, J = 12Hz, CH_{A}=CH_{B}), 7.10-7.55 (7H, m, ArH). m/e 284 and 282 (M⁺).

### Step B 3-Bromo-5-hydroxymethyl-dibenzo[a,d] cycloheptene

To a solution of 3-bromo-5-methylene-dibenzo[a,d] cycloheptene (3.8g) in dry tetrahydrofuran (20ml), under an inert atmosphere, was added 9-borabicyclo [3,3,1]nonane (30ml of a 0.5 molar solution in tetrahydrofuran) at ambient temperature and the reaction mixture was refluxed for 3h. After cooling in an ice bath the reaction was quenched by the slow addition of 2N sodium hydroxide solution (100ml) and 30% hydrogen peroxide (20ml). After stirring vigorously at 0°C for 45 minutes the reaction mixture was allowed to warm to room temperature over a 2 hour period then extracted with diethyl ether (3 x 200ml). The combined organic layers were washed with brine (1 x 100ml), dried (Na₂SO₄) filtered and concentrated in vacuo to leave a residue which was purified by chromatography on flash silica with 30% ethyl acetate in hexane as eluent to give the title compound (2.3g) as a colourless oil. δ (CDCl₃, 360MHz) 3.83 (2H, d, J = 7.9Hz, CH₂OH), 4.13 (1H, t, J = 7.9Hz, CH-CH₂OH), 6.78 (1H, d, J = 12Hz, CH_{A}=CH_{B}), 6.87 (1H, d, J = 12Hz, CH_{A}=CH_{B}), 7.17-7.50 (7H, m, ArH). m/e 302 and 300 (M⁺).

### Step C 3-Bromo-5-hydroxy-dibenzo[a,e]cyclooctene and 3-bromo-6-hydroxy-dibenzo[a,e]cyclooctene

To a solution of 3-bromo-5-hydroxymethyldibenzo[a,d]cycloheptene (2.2g) in dry dichloromethane (50ml) was added p-toluene sulphonyl chloride (1.7g), pyridine (1.2g) and 4-dimethylamino pyridine (100mg). The reaction mixture was refluxed for 14h, cooled, washed with water (2 x 40ml), dried (Na₂SO₄), filtered and concentrated to leave a residue. This was purified by chromatography on flash silica to give a colourless oil (4.5g) which was dissolved in glacial acetic acid (50ml) with anhydrous sodium acetate (1.6g). The reaction mixture was heated at reflux for 24h then concentrated to leave a residue which was dissolved in 75% aqueous methanol (50ml) and treated with potassium hydroxide (4g). After 14h the solvent was removed in vacuo and the residue partitioned between water (50ml) and diethyl ether (3 x 50ml). The combined organic layers were dried (Na₂SO₄), filtered and concentrated in vacuo. Chromatography of the residue on flash silica with 15% ethyl acetate in hexane as eluent gave the less polar isomer 3-bromo-5-hydroxy-dibenzo[a,e]cyclooctene (0.8g) as a colourless oil. δ (360MHz, CDCl₃) 3.25 (1H, dd, J = 14.1 and 9.7Hz, CH_{A}H_{B}-CHOH), 3.48 (1H, dd, J = 14.1 and 6.1Hz, CH_{A}H_{B}-CHOH), 5.26 (1H, dd, J = 9.7 and 6.1Hz, CH_{A}H_{B}-CHOH), 6.75 (1H, d, J = 12.3Hz, CH_{C}=CH_{D}), 6.85 (1H, d, J = 12.3Hz, CH_{C}=CH_{D}), 6.94-7.28 (6H, m, ArH), 7.63 (1H, d, J = 2Hz, CH_{A}H_{B}-CH(OH)-C=CH-CBr=CH). m/e 302 and 300 (M⁺); and the more polar isomer 3-bromo-6-hydroxy-dibenzo[a,e]cyclooctene (0.26g) as a colourless oil. δ (360MHz, CDCl₃) 3.26 (1H, dd, J = 13.9 and 10.1Hz, CH_{A}H_{B}-CHOH), 3.42 (1H, dd, J = 13.9 and 6.3Hz, CH_{A}H_{B}-CHOH), 5.27 (1H, dd, J = 10.1 and 6.3Hz, CH_{A}H_{B}-CHOH), 6.73 (1H, d, J = 12.3Hz, CH_{C}=CH_{D}), 6.89 (1H, d, J = 12.3Hz CH_{C}=CH_{D}), 6.93-7.41 (7H, m, ArH). m/e 302 and 300 (M⁺).

### Step D 3-Bromo-5-oxo-dibenzo[a,e]cyclooctene

To a solution of 3-bromo-5-hydroxy-dibenzo[a,e] cyclooctene (0.75g) in dry dichloromethane (60ml) was added pyridinium dichromate (2.1g) and crushed 4A molecular sieves (1.3g). After stirring at room temperature for 14h, diethyl ether (150ml) was added and the reaction mixture was filtered through a plug of celite. The solvent was removed and the residue purified by chromatography on flash silica with 5% ethyl acetate in hexane as eluent to give, as a colourless solid, the title compound (0.7g,). δ (360MHz, CDCl₃) 4.04 (2H, s, CH₂-C=O), 6.95 (1H, d, J = 12.8Hz, CH_{A}=CH_{B}), 7.09 (1H, d, J = 12.8Hz, CH_{A}=CH_{B}), 7.22-7.59 (6H, m, ArH), 8.36 (1H, d, J = 2.2Hz, C(O)-C=CH-CBr).

### Step E 8-Bromo-13-hydroxy-6-methyl-5,6,11,12-tetrahydrodibenzo[a,e]cycloocten-6,11-imine

To a solution of 3-bromo-5-oxo-dibenzo[a,e] cyclooctene (0.68g) in dry diethyl ether (30ml) at 0°C under an atmosphere of nitrogen was added methyl magnesium bromide (1.6ml of a 3 molar solution in diethyl ether). After 2h at room temperature the reaction mixture was cooled to 0°C, quenched by the dropwise addition of saturated ammonium sulphate solution (20ml) and extracted with diethyl ether (3 x 30ml). The combined organic layers were dried (Na₂SO₄), filtered and evaporated to leave an oil which was dissolved in dry dichloromethane (10ml) and added to a solution containing dichloroacetic acid (3.1ml), sodium acetate (2.1g) and hydroxylamine hydrochloride (1.75g) in dichloromethane (40ml), which had been prepared 2 hours previously. The reaction mixture was stirred at room temperature for 6h then refluxed for a further 6h. After cooling, 2N potassium hydroxide solution was added until the pH was 14 and the two phase mixture was stirred vigorously for 30 minutes. The organic layer was separated, washed with water (1 x 40ml) and brine (1 x 40ml), dried (Na₂SO₄) filtered and concentrated in vacuo. The crude residue was purified by chromatography on flash silica with 40% ethyl acetate in hexane as eluent, to give the title compound as a white foam (0.147g). The nmr spectrum (360MHz, CDCl₃) showed a mixture of two atropisomers.

### Step F 8-Bromo-6-methyl-5,6,11,12-tetrahydrodibenzo [a,e]cycloocten-6,11-imine hydrochloride

To a solution of 8-bromo-13-hydroxy-6-methyl-5,6,11,12-tetrahydrodibenzo[a,e]cycloocten-6,11-imine (0.15g) in glacial acetic acid (6ml) was added zinc dust (0.3g). The reaction mixture was heated at 65°C under an inert atmosphere for 6h then filtered and concentrated to leave a residue which was partitioned between dichloromethane (30ml) and dilute sodium hydroxide solution (30ml). The organic layer was separated, dried (Na₂SO₄), filtered and evaporated in vacuo to leave a residue which was dissolved in 5 molar hydrogen chloride in ethyl acetate (20ml). The solvent was evaporated to leave a solid which was recrystallised from ethyl acetate/diethyl ether. The title compound (0.055g, m.p. 186-189°C) was collected by filtration and dried under high vacuum. δ (DMSO, 360MHz) 1.89 (3H, s, CH₃), 3.13 (1H, d, J = 16.5Hz, PhCH_{A}H_{B}-C(NH) CH₃), 3.30 (1H, dd, J = 16.6 and 5.0Hz, PhCH_{C}H_{D}-C(NH)H_{E}), 3.68 (1H, d, J = 16.6Hz, PhCH_{C}H_{D}-C(NH)H_{E}), 3.82 (1H, d, J = 16.5Hz, PhCH_{A}H_{B}-C(NH)CH₃), 5.19 (1H, d, J = 5.0Hz, PhCH_{C}H_{D}-C(NH)H_{E}), 6.90-7.00 (4H, m, ArH), 7.22 (1H, d, J = 8.0Hz, C(NH)H_{E}-C=CH_{F}-CH_{G}=CBr-CH_{H}), 7.36 (1H, dd, J = 8.0 and 1.7Hz, C(NH)H_{E}-C= CH_{F}-CH_{G}=CBr-CH_{H}), 7.51 (1H, d, J = 1.7Hz, C(NH)H_{E}-C=CH_{F}-CH_{G}=CBr-CH_{H}). n.O.e's observed from CH₃ to H_{H} and H_{E} to H_{F}.

### EXAMPLE 7

### 5-Endo,6-dimethyl-5,6,11,12-tetrahydrodibenzo[a,e] cycloocten-6,11-imine acetate

### Step A 5-Methyl-6-oxo-dibenzo[a,e]cyclooctene

To a stirred solution of disopropylamine (3.61ml) in anhydrous tetrahydrofuran at 0°C under nitrogen, was added a solution of n-butyllithium (8.72ml of a 2.5M solution in hexane). The resulting solution was warmed to room temperature, then cooled to -60°C and a solution of 5-oxo-dibenzo[a,e]cyclooctene (Example 1E, 4.0g, 18.2mmol) in anhydrous tetrahydrofuran (60ml) added slowly. The reaction mixture was warmed to 0°C, then iodomethane (2.27ml) added and the solution stirred at room temperature for 14h. The reaction was quenched with saturated ammonium sulphate solution (80ml) and ammonium hydroxide solution (4M, 40ml). After stirring for 30 minutes the reaction mixture was extracted with diethyl ether (1 x 50ml), the ethereal layer was washed successively with water (4 x 20ml) then brine (1 x 20ml) and dried (Na₂SO₄). The solvent was removed in vacuo to give an oil which was separated by silica gel chromatography using 5% ethyl acetate in petroleum ether as eluent to give a colourless oil which was crystallised from hexane to give the title compound (0.91g), mp 74-76°C δ (CDCl₃ 360MHz,) δ 1.62 (3H, d, CH-CH₃, J = 6.7Hz), 4.42 (1H, q, J = 6.7Hz CH-CH₃), 7.08 (2H, s, CH = CH), 7.17-7.51 (7H, m, ArH), 8.31 (1H, dd, J = 1.4Hz and 8.1Hz, ArH).

### Step B 5-Hydroxy-5,6-dimethyl-dibenzo[a,e] cyclooct-11-ene

To a stirred solution of 5-methyl-6-oxo-dibenzo[a,e]cyclooctene (0.1g) in anhydrous diethyl ether (10ml) under nitrogen at 0°C was added methyl lithium (0.43ml of a 1.4M solution in diethyl ether). After stirring for approximately 30 minutes water (10ml) was added. The phases were separated and the ethereal layer was washed with water (2 x 10ml), dried (Na₂SO₄) and the solvent removed in vacuo to give the title compound as a colourless solid (0.104g). mp 86-87°C. δ 1.43 (CDCl₃ 360MHz,) (3H, s, C-CH₃), 1.44 (3H, d, J = 7.0Hz, -CH-CH₃), 2.03 (1H, s, -OH), 4.07 (1H, q, J = 7.0Hz, -CH-CH₃), 6.75 (1H, d, J = 11.8Hz, CH_{A}=CH_{B}), 6.96-7.21 (8H, m, ArH and CH_{A}=CH_{B}), 7.41 (1H, d, J = 7.5Hz, ArH), 7.85 (1H, d, J = 7.9Hz, ArH) m/e 250 (M⁺).

### Step C 13-Hydroxy-5-endo,6-dimethyl-5,6,11,12-tetrahydrodibenzo[a,e]cycloocten-6,11-imine

To a stirred solution of dichloroacetic acid (3.71ml, 45mmol) in dry dichloromethane (3.7ml) at 0°C was added anhydrous sodium acetate (2.46g) and hydroxylamine hydrochloride (2.09g). The resulting slurry was stirred at room temperature for 1.5h, additional dry dichloromethane (30ml) was added, and after a further 1.5h a solution of 5-hydroxy-5,6-dimethyl-dibenzo[a,e]cyclooct-11-ene (750mg) in dry dichloromethane (8ml) was introduced. The mixture was stirred at room temperature for 1h, then trifluoroacetic acid (3.7ml) added and the resulting solution stirred for a further 1.5h. The reaction was quenched with water (30ml) and basified to pH 11 with concentrated ammonia solution. The phases were separated and the organic layer washed with brine (10ml) and dried over anhydrous sodium sulphate. Removal of the solvent gave a foam which was triturated with 20% ethyl acetate in petroleum ether to give, after filtration, the title compound (0.14g). mp 124-127°C. δ (CDCl₃ 360MHz, )1.39 (3H, d, J = 7.4Hz, CH-CH₃), 1.62 (3H, s, C-CH₃), 2.56 (1H, dd, J = 5.8Hz and 15.5Hz, H_{A}CH_{B}-CH_{C}), 4.28 (1H, q, J = 7.4Hz, CH-CH₃) 4.32 (1H, d, J = 15.5Hz, H_{A}CH_{B}-CH_{C}), 4.62 (1H, d, J = 5.8Hz, H_{A}CH_{B}CH_{C}), 6.9-7.1 (8H, m, ArH). m/e 265 (M⁺).

### Step D 5-Endo,6-dimethyl-5,6,11,12-tetrahydrodibenzo [a,e]cycloocten-6,11-imine acetate

A mixture of 3-hydroxy-5,6-dimethyl-5,6,11,12-tetrahydrodibenzo[a,e]cycloocten-6,11-imine (0.5g), zinc dust (75mg) and glacial acetic acid (0.40ml) was heated at 60-65°C under nitrogen for 26 hours. The mixture was then filtered, and the filtrate evaporated under vacuum to give an oil. This was partitioned between diethyl ether (10ml) and concentrated ammonium hydroxide solution (10ml) and the ethereal layer was washed with saturated brine (10ml), then dried over anhydrous sodium sulphate. Removal of the solvent gave a solid which was triturated with diethyl ether to give the title compound (0.35g) as the acetate salt, mp 112-119°C (dec). δ (CDCl₃ 360MHz,) 1.47 (3H, d, J = 7.5Hz, CH-CH₃), 1.70 (3H, s, C-CH₃), 2.03 (3H, s, CH₃CO₂H), 3.11 (1H, dd, J = 6.2Hz and 15.5Hz, H_{A}CH_{B}-CH_{C}), 3.55 (1H, q, J = 7.5Hz, CH-CH₃), 3.61 (1H, d, J = 15.5Hz, H_{A}CH_{B}-CH_{C}), 4.71 (1H, d, J = 6.2Hz, H_{A}CH_{B}-CH_{C}), 6.79-6.98 (8H, m, ArH). m/e 249 (M⁺).

### EXAMPLE 8

### Resolution of 6-methyl-5,6,11,12-tetrahydrodibenzo [a,e]cycloocten-6,11,-imine

### Step A Preparation of Diastereoisomers of N(L-phenylalanyl)-6-methyl-5,6,11,12-tetrahydrodibenzo [a,e]cycloocten-6,11-imine

To a solution of tert-butyloxycarbonyl-L-phenylalanine (0.397g) in dry dichloromethane (5ml) at 0°C was added diisopropylethylamine (0.26ml) and bis (2-oxo-oxazolidinyl) phosphinyl chloride (0.381g) and the mixture stirred at 0°C for ten minutes. A solution of 6-methyl-5,6,11,12-tetrahydrodibenzo [a,e]cycloocten-6,11-imine (0.350g) in dichloromethane (2ml) was added in one portion, followed by diisopropyl ethylamine (0.26ml). The reaction mixture was allowed to warm to room temperature and stirred overnight. The solvents were evaporated and the residue partitioned between ethyl acetate (5ml) and water (5ml). The organic layer was washed with saturated aqueous sodium bicarbonate and water, then dried and evaporated to give a crude mixture of diastereoisomers of N-(tert-butoxy-L-phenylalanyl)-6-methyl-5,6,11,12-tetrhydrodibenzo[a,e]-cycloocten-6,11 -imine (0.350g). To this crude mixture was added trifluoroacetic acid (2ml) and the solution stirred for 15 minutes then evaporated to dryness. The residue was dissolved in ethyl acetate (5ml) and water (5ml) and 2N aqueous sodium hydroxide (5ml) were added. After shaking the aqueous layer was allowed to separate and the discarded. The organic layer was washed with water and brine, then dried and evaporated to give a crude mixture of diastereoisomers of N(L-phenylalanyl)-6-methyl-5,6,11,12-tetrahydrodibenzo[a,e]-cycloocten-6, 11-imine. These were separated by chromatography on silica using 2% isopropyl alcohol in chloroform as eluent to give (i) Diastereoisomer A (95mg). δ (CDCl₃, 360MHz) 2.13 (3H, s, CH₃), 2.68 (1H, d, J = 15.6Hz), 2.87 (3H, m), 3.29 (1H, m), 3.94 (1H, m), 4.11 (1H, d, J = 15.6Hz), 5.45 (1H, m), 6.6-7.3 (13H, m, ArH); (ii) Diastereoisomer B (105mg). δ (CDCl₃, 360MHz), 2.08 (3H, s, CH₃), 2.86 (2H, m), 3.06 (2H, m), 3.55 (1H, d, J = 15.7Hz), 4.04 (1H, m), 4.22 (1H, d, J = 16.0Hz), 5.10 (1H, m), 6.77-7.25 (13H, ArH).

### Step B Preparation of (+) 6-methyl-5,6,11,12-tetrahydrodibenzo[a,e]cycloocten-6,11-imine hydrochloride

To a solution of diastereoisomer A (Step A, 90mg) in ethyl alcohol (3ml) was added diisopropyl ethylamine (96»l) and phenyl isothiocyanate (74»l) and the solution was stirred for 30 minutes. The solvents were evaporated and the residue dissolved in trifluoroacetic acid (2ml), warmed to 40°C and allowed to stand for 1 hr. The solvent was removed and the residue partitioned between ether (5ml) and water (3ml). The organic layer was discarded and the aqueous treated with 1N sodium hydroxide to pH 12 and extracted with ethyl acetate. The organic layer was washed with water, dried and evaporated to give crude (+) 6-methyl-5,6,11,12-tetrahydrodibenzo[a,e]cycloocten-6,11-imine which was purified by column chromatography using 5% methanol in chloroform as eluent. This product was dissolved in a little ethyl acetate and treated with 5M hydrogen chloride in ethyl acetate to give the hydrochloride salt, which was freeze dried from water, to give (+)-6-methyl-5,6,11,12-tetrahydrodibenzo[a,e]-cycloocten-6,11-imine hydrochloride (36mg) mp 280-5°C. The ¹H nmr spectrum was identical with the racemate (Example 1I).

### Step C Preparation of (-)-6-methyl-5,6,11,12-tetrahydrodibenzo[a,e]cycloocten-6,11-imine hydrochloride

Using the above procedure (Step B) diastereoisomer B (Step A, 100mg) for diastereoisomer A gave (-)-6-methyl-5,6,11,12-tetrahydrodibenzo [a,e]-cycloocten-6,11-imine hydrochloride (50mg). mp 282-5°C. The ¹H nmr spectrum was identical with the racemate (Example 1I).

### EXAMPLE 9

### 6-Methyl-11-n-butyl-5,6,11,12-tetrahydrodibenzo[a,e]cycloocten-6,11-imine hydrochloride

### Step A Preparation of 6-methoxylamino-6-methyl-5,6-dihydrodibenzo[a,e]cyclooctene

To a solution of dichloroacetic acid (19.4g) in dry dichloromethane (25ml) was added anhydrous sodium acetate (8.2g) with cooling and stirring. The mixture was stirred until the solid had completely dissolved, then a further 15ml dichloromethane was added followed by methoxylamine hydrochloride (8.35g). After 30 mins stirring a solution of 6-methyl-6-hydroxy-5,6-dihydrodibenzo[a,e]cyclooctene (2.36g) in dichloromethane (10ml) was added and the mixture stirred at room temperature for 4 hours. The mixture was cooled in ice, treated with 2N sodium hydroxide to pH 12 and the organic layer separated, washed with water, dried and evaporated to give a clear oil. This was chromatographed on silica using 20% ethyl acetate/in hexane as eluent to give 6-methoxylamine-6-methyl-5,6-dihydrodibenzo[a,e] cyclooctene (2.21g). mp 48-50°C.

### Step B Preparation of N-methoxy-6-methyl-5,6,11,12-tetrahydrodibenzo[a,e]cycloocten-6,11-imine

To a solution of the product from Step A (1.90g) in 1:9 dimethylsulfoxide/toluene (20ml) at room temperature was added potassium tert-butoxide (0.80g) in one portion. The suspension was warmed to 55°C with stirring for 3 minutes under nitrogen. The mixture was cooled, diluted with one volume of diethyl ether, washed with water (3 x 20ml), dried and evaporated. Crystallisation from ether/hexane gave N-methoxy-6-methyl-5,6,11,12-tetrahydrodibenzo [a,e]cycloocten-6,11-imine (1.28g). mp 85-8°C.

### Step C Preparation of 6-methyl-11-n-butyl-5,6,11,12-tetrahydrodibenzo[a,e]cycloocten-6,11-imine hydrochloride

To a solution of the product from Step B (0.132g) in anhydrous ether (3ml) under nitrogen at -78°C was added a solution of n-butyl lithium (0.3ml of a 1.47M solution in hexane). The solution was allowed to warm to room temperature and stirred for 4 hrs. Water (2ml) was cautiously added and the aqueous layer separated and discarded. The organic layer was dried and evaporated, then chromatographed on silica using 1:1 ethyl acetate/hexane as eluent. Further chromatography using 1:4 ethyl acetate/hexane as eluent afforded pure 6-methyl-11-n-butyl-5,6,11,12-tetrahydrodibenzo[a,e]cycloocten-6,11-imine (0.046g). This was dissolved in ethyl acetate (1ml) and 5M hydrogen chloride in ethyl acetate (0.5ml) was added. Evaporation and trituration with ether gave the required 6-methyl-11-n-butyl-5,6,11,12-tetrahydrodibenzo[a,e]cycloocten-6,11-imine hydrochloride (0.045g). mp 230-234°C.

### EXAMPLE 10

### 3-Bromo-6-methyl-5,6,11,12-tetrahydrodibenzo[a,e] cycloocten-6,11-imine hydrochloride

### Step A 3-Bromo-6-oxo-dibenzo[a,e]cyclooctene

To a solution of 3-bromo-6-hydroxy-dibenzo[a,e] cyclooctene (Example 6C) (2g) in dry dichloromethane (60ml) was added pyridinium dichromate (4.9g) and crushed 4A molecular sieves (3.2g). After stirring at room temperature for 5h, diethyl ether (100ml) was added and the reaction mixture was filtered through a plug of celite. The solvent was removed in vacuo and the residue then dissolved in dichloromethane (100ml), washed with water (100ml), dried (Na₂SO₄), filtered and concentrated in vacuo to give the title compound as an oil (1.6g). δ (360MHz, CDCl₃) 4.04 (2H, s, CH₂-C=O), 6.95 (1H, d, J = 12.8Hz, ArCH_{A}=CH_{B}Ar), 7.08 (1H, d, J = 12.8Hz, ArCH_{A}=CH_{B}Ar), 7.23-7.53 (6H, m, aromatics) and 8.19 (1H, d, J = 8.2Hz, C(O)-C=CH-CH=CH-CH). m/e 300 and 298 (M⁺)

### Step B 3-Bromo-13-hydroxy-6-methyl-5,6,11,12-tetrahydrodibenzo[a,e]cycloocten-6,11-imine

To a solution of 3-bromo-6-oxo-dibenzo[a,e] cyclooctene (1.5g) in dry diethyl ether (50ml) at 0°C under an atmosphere of nitrogen was added methyl magnesium bromide (5.0ml of a 3 molar solution). After 4h at room temperature the reaction mixture was cooled to 0°C, quenched by the dropwise addition of saturated ammonium sulphate solution (30ml) and extracted into diethyl ether (3x50ml). The combined organic layers were dried (Na₂SO₄), filtered and evaporated to leave an oil (1.6g) which was dissolved in dry dichloromethane (12ml) and added to a solution containing dichloroacetic acid (6.3ml), sodium acetate (4.1g), and hydroxylamine hydrochloride (3.47g) in dichloromethane (40ml), which had been prepared 3 hours previously. The reaction mixture was stirred at room temperature for 14h and then refluxed for a further 7h. After cooling, 2N potassium hydroxide solution was added until the pH was 14 and the two phase mixture was stirred vigorously for 30 minutes. The organic layer was separated, washed with water (1x60ml) and then brine (1x60ml), dried (Na₂SO₄), filtered and concentrated in vacuo. The crude residue was purified by chromatography on flash silica with 15% ethyl acetate in dichloromethane as eluant to give the title compound (0.195g), m.p.171-176°C. The NMR spectrum (360MHz, CDCl₃) showed a mixture of two atropisomers. m/e 331 and 329(M⁺).

### Step C 3-Bromo-6-methyl-5,6,11,12-tetrahydrodibenzo[a,e]cycloocten-6,11-imine hydrochloride

To a solution of 3-bromo-13-hydroxy-6-methyl-5,6,11,12-tetrahydrodibenzo[a,e]cycloocten-6,11-imine (0.190g) in glacial acetic acid (10ml) was added zinc dust (0.40g). The reaction mixture was heated at 65°C under an atmosphere of nitrogen for 6h then filtered and concentrated in vacuo to leave a residue which was partitioned between dichloromethane (50ml) and 1N sodium hydroxide solution (50ml). The organic layer was separated, dried (Na₂SO₄), filtered and evaporated to leave a residue which was dissolved in 5 molar hydrogen chloride in ethyl acetate (10ml). The solvent was evaporated to leave a solid which was recrystallised from ethyl acetate-diethyl ether. The title compound was collected by filtration and dried under high vacuum (0.047g), m.p. 274°C. δ (360MHz) 2.15 (3H, s, CH₃), 2.85 (1H, d, J = 16.5Hz, CH_{A}H_{B}-C(CH₃)), 3.16 (1H, dd, J = 16.8 and 5.2Hz, CH_{C}H_{D}-CH_{E} NH), 4.12 (1H, d, J = 16.8Hz, CH_{C}H_{D}-CH_{E} NH),4.32 (1H, d, J = 16.5Hz, CH_{A}H_{B}-C(CH₃)), 5.30 (1H, d, J = 5.2Hz, CH_{C}H_{D}-CH_{E} NH), 6.73 (1H, d, J = 8.1Hz, CH_{F}= CH_{G}-C(Br)-CH_{H}), 6.96 (1H, d, J = 1.9Hz, CH_{F}= CH_{G}-C(Br)-CH_{H}), and 7.03 - 7.21 (5H, m, aromatics). Irradiation of H_{D} (δ4.1) gave a n.O.e to H_{F}(δ 6.73) and irradiation or H_{B}(δ 4.32) gave a n.O.e to H_{H}(δ 6.96). m/e, found 313.0442; C₁₇H₁₆NBr requires 313.0466. Found C,56.9; H,5.0; N,3.90. C₁₇H₁₇NBr.HCl.0.5H₂O requires C, 56.8; H, 5.0; N, 3.90%.

### EXAMPLE 11

### 10-Chloro-6-methyl-5,6,11,12-tetrahydrodibenzo [a,e]cycloocten-6,11-imine hydrochloride

### Step A 1-Chloro-5-methylene-dibenzo[a,d]cycloheptene

A solution of 1-chloro-dibenzo[a,d]cyclohepten-5-one (13.0g) in anhydrous tetrahydrofuran (80.0ml) was cooled to 0°C and methyllithium (35.5ml of a 1.6 molar solution) was added by syringe. After 1h, water (60ml) was added dropwise and the reaction mixture extracted with diethyl ether (3x100ml). The combined organic layers were dried over sodium sulphate, filtered and concentrated in vacuo to give a pale yellow oil (13.02g). This was dissolved in dry dichloromethane (80ml) with dichloroacetic acid (2.5ml) and refluxed for 9h. The mixture was cooled, washed with saturated sodium hydrogen carbonate solution (1x120ml) and brine (1x120ml), dried (Na₂SO₄), filtered and concentrated in vacuo to give the title compound as an oil (9.1g). δ (360MHz, CDCl₃) 5.25 (1H, d, J = 1.4Hz, CH_{A}H_{B}), 5.26 (1H, d, J = 1.4Hz, CH_{A}H_{B}), 6.98 (1H, d, J = 11.8Hz, ArCH_{C}-CH_{D}Ar), 7.20 (1H, d, J = 11.8Hz, ArCH_{C}-CH_{D}Ar) and 7.20-7.38 (7H, m, aromatics). m/e 238 (M⁺).

### Step B 1-Chloro-5-hydroxymethyl-dibenzo[a,d]cyclo heptene

To a solution of 1-chloro-5-methylene-dibenzo[a,d] cycloheptene (9.0g) in dry tetrahydrofuran (60ml), under an inert atmosphere, was added 9-borabicyclo[3, 3,1]nonane (80.5ml of a 0.5 molar solution in THF) at ambient temperature and the reaction mixture was then refluxed for 3h. After cooling, the reaction was quenched by the slow addition of 2N sodium hydroxide solution (180ml) and 30% hydrogen peroxide (36ml). After stirring vigorously at 0°C for 45 minutes the reaction mixture was allowed to warm to room temperature over a 2h period then extracted into diethyl ether (3x250ml). The combined organic layers were washed with brine (1x500ml), dried (Na₂SO₄), filtered and concentrated in vacuo to leave a residue which was purified by chromatography on flash silica with 40% ethyl acetate in hexane as eluent to give the title compound (8.3g) as a colourless oil. δ (360MHz, CDCl₃) 3.87 (2H, m, CH-CH₂OH), 4.21 (1H, t, J = 7.9Hz, CH-CH₂OH), 7.02 (1H, d, J = 11.9Hz, CH_{A}=CH_{B}) and 7.17-7.46 (8H, m, CH_{A}=CH_{B} and 7 aromatic protons). m/e 258 and 256 (M⁺).

### Step C 1-Chloro-5-hydroxy-dibenzo[a,e]cyclooctene and 1-Chloro-6-hydroxy-dibenzo[a,e]cyclooctene

To a solution of 1-chloro-5-hydroxymethyl-dibenzo [a,d]cycloheptene (8.3g) in dichloromethane (80ml) was added p-toluenesulphonyl chloride (6.3g), pyridine (5.11g), and 4-dimethylamino pyridine (150mg). The reaction mixture was refluxed for 15h, cooled, washed with water (2x80ml), dried (Na₂SO₄), filtered and concentrated in vacuo to leave an oil which was purified by chromatography on flash silica with 15% ethyl acetate in hexane as eluent. The purified tosylate was dissolved in glacial acetic acid (100ml) with anhydrous sodium acetate (4.4g) and the solution refluxed for 15h . The mixture was concentrated in vacuo to leave a residue which was partitioned between dichloromethane (3x100ml) and water (100ml). The combined organic layers were dried (Na₂SO₄), filtered and concentrated in vacuo to leave a residue which was dissolved in 75% aqueous methanol (160ml) and treated at 0°C with potassium hydroxide (10g). After 14h the reaction mixture was concentrated in vacuo. Chromatography of the residue on flash silica with 15% ethyl acetate in hexane as eluent gave the less polar isomer, 1-chloro-5-hydroxydibenzo[a,e]cyclooctene (1.5g), m.p. 158 - 160°C. δ (360MHz, CDCl₃) 3.12 (1H, dd, J = 1.51 and 10.2Hz, CH_{A}H_{B}-CHOH), 3.63 (1H, dd, J = 15.1 and 6.9Hz, CH_{A}H_{B}-CHOH), 5.57 (1H, dd, J = 10.2 and 6.9Hz, CH_{A}H_{B}-CHOH), 6.74 (1H, d, J = 11.9Hz, ArCH_{D}=CH_{E}-Ar), 7.03 (1H, d, J = 11.9Hz, ArCH_{D}=CH_{E}Ar) and 7.07 - 7.46 (7H, m, aromatics). m/e 256 (M⁺). i.r.(νₘₐₓ,nujol) 3350 - 3100 cm⁻¹ (br,OH). Found: C,74.5; H, 5.1. C₁₆H₁₃Cl0 requires: C,74.9; H,5.1%. Further elution gave the more polar isomer. 1-chloro-6-hydroxy-dibenzo[a,e]cyclooctene (0.47g), m.p. 144 - 146°C. δ (360MHz, CDCl₃) 3.39 (2H, m, CH₂-CHOH), 5.13 (1H, m, CH₂-CHOH), 6.77 (1H, d, J = 11.9Hz, Ar-CH_{A}=CH_{B}), 7.02 (1H, d, J = 11.9Hz, ArCH_{A}=CH_{B}) and 7.07 - 7.38 (7H, m, aromatics). m/e 256 (M⁺). i.r (νₘₐₓ,nujol) 3300-3100cm⁻¹ (br,OH). Found: C,74.8; H,5.2. C₁₆H₁₃Cl0 requires: C,74.9; H,5.1%.

### Step D 1-Chloro-5-oxo-dibenzo[a.e]cyclooctene

To a solution of 1-chloro-5-hydroxy-dibenzo[a,e] cyclooctene (1.5g) in dry dichloromethane (60ml) was added pyridinium dichromate (5.0g) and crushed 4A molecular sieves (2.5g). After stirring at room temperature for 3h, diethyl ether (150ml) was added and the reaction mixture was filtered through a plug of celite. The solvent was removed in vacuo and the residue purified by chromatography on flash silica with 10% ethyl acetate in hexane as eluent to give the title compound (1.35g), m.p. 151-153°C. δ (360MHz, CDCl₃) 4.09 (2H, s, CH₂-C=O), 7.06 (1H, d, J = 11.9Hz, ArCH_{A}=CH_{B}Ar), 7.18 (1H, d, J = 11.9Hz, ArCH_{A}=CH_{B}Ar), 7.20 - 7.30 (5H, m, aromatics), 7.50 (1H, dd, J = 7.8 and 1.4Hz, CH=CCl) and 7.86 (1H, dd, J = 8.0 and 1.3Hz, CH=C-C=O). m/e 254 (M⁺). i.r. (νₘₐₓ,nujol) 1670 cm⁻¹ (CO). Found: C,75.1; H,4.6. C₁₆H₁₁Cl0 requires: C,75.4; H,4.4%.

### Step E 10-Chloro-13-hydroxy-6-methyl-5,6,11,12 -tetrahydrodibenzo[a,e]cyclooctene-6-,11-imine

To a solution of 1-chloro-5-oxo-dibenzo[a,e] cyclooctene (1.3g) in dry diethyl ether (60.0ml) at 0°C under an atmosphere of nitrogen was added methyl magnesium bromide (4.0ml of a 3 molar solution). After 3h at room temperature the mixture was cooled to 0°C, quenched dropwise with saturated ammonium sulphate solution (20.0ml) and extracted with diethyl ether (3x50ml). The combined organic layers were dried (Na₂SO₄), filtered and evaporated to leave an oil (1.1g), which was dissolved in dry dichloromethane (15ml) and added to a solution containing dichloroacetic acid (5.6ml), sodium acetate (3.69g) and hydroxylamine hydrochloride (3.12g) in dichloromethane (40ml). The reaction mixture was stirred at room temperature for two hours then refluxed for a further 24h. After cooling, 2N potassium hydroxide solution was added until the pH was 14 and the two phase mixture was stirred vigorously for 30 minutes. The organic layer was separated, washed with water (1x50ml) and brine (1x50ml), dried (Na₂SO₄), filtered and concentrated in vacuo. The crude product was purified by chromatography using flash silica with 5% ethyl acetate in dichloromethane as eluent to give the title compound (0.13g), m.p. 162-164°C. The NMR spectrum (360MHz, CDCl₃) showed a mixture of two atropisomers. m/e 287 and 285 (M⁺).

### Step F 10-Chloro-6-methyl-5,6,11,12-tetrahydrodibenzo[a,e]cyclooctene-6,11-imine hydrochloride

To a solution of 10-chloro-13-hydroxy-6-methyl-5,6,11,12-tetrahydrodibenzo[a,e]cyclooctene-6,11-imino (0.13g) in glacial acetic acid (5ml) was added zinc dust (0.26g). The reaction mixture was heated at 70°C for 8h, then cooled, filtered and concentrated in vacuo to leave a residue which was partitioned between dichloromethane (30ml) and dilute sodium hydroxide solution (30ml). The organic layer was separated, dried (Na₂SO₄), filtered and concentrated in vacuo. The residue was purified by chromatography using flash silica with 3% methanol in dichloromethane as eluent. The product was dissolved in 5 molar hydrogen chloride in ethyl acetate (15ml) and the solvent was evaporated to give the title compound (0.06g), m.p. 196-198°C. δ (360MHz, DMSO) 1.91 (3H, s, CH₃), 3.09 (1H, d, J = 16.4Hz, CH_{A}H_{B}-C(CH₃), 3.35 (1H, dd, J = 16.7 and 5.1Hz, CH_{C}H_{D}-CH_{E}NH), 3.78 (1H, d, J = 16.7Hz, CH_{C}H_{D}-CH_{E}NH), 3.95 (1H, d, J = 16.4Hz, CH_{A}H_{B}-C(CH₃)), 5.26 (1H, d, J = 5.1Hz) and 6.87 - 7.24 (7H, m, aromatics). Irradiation of H_{E} (δ 5.26) gave no n.o.e. to the aromatic protons and irradiation of H_{C} (δ 3.35) did give a n.O.e to the aromatic protons. m/e, Found: 269. 0968, C₁₇H₁₆ClN requires: 269. 0971. Found: C,64.4; H,5.6; N,4.3. C₁₇H₁₃ClN.HCl.0.5H₂0 requires: C,64.8; H,5.8; N,4.4%.

### EXAMPLE 12

### 2-Methoxy-6-methyl-5,6,11,12-tetrahydrodibenzo [a,e]cycloocten-6,11-imine hydrochloride

### Step A 2-Methoxy-5-methylene-dibenzo[a,d]cycloheptene

A solution of 2-methoxy-dibenzo[a,d]cyclohepten-5-one (20g) in anhydrous tetrahydrofuran (200ml) was cooled to 0°C and methyllithium (55.8ml of a 1.6 molar solution) was added by syringe. After 45 minutes, water (70ml) was added dropwise and the reaction mixture extracted with diethyl ether (3x100ml). The combined organic phases were dried over sodium sulphate, filtered and concentrated in vacuo to give a colourless oil (18.8g). This was dissolved in dichloromethane (120ml) with dichloroacetic acid (2.5ml) and stirred at room temperature for 2h, washed with saturated sodium hydrogen carbonate solution (100ml), dried (Na₂SO₄), filtered and concentrated in vacuo. The residue was purified by chromatography using flash silica with 5% ethyl acetate in hexane as eluent to give the title compound as a colourless solid (12.95g, m.p. 131-133°C). δ (360MHz, CDCl₃) 3.8 (3H, s, OCH₃), 5.21 (2H, 2d, J = 1.95Hz, =CH₂), δ 6.75 (1H, d, J = 11.8Hz, Ar-CH_{A}=CH_{B}Ar), 6.76 (1H, d, J = 2.7Hz, CH=(COCH₃)-CH), 6.90 (1H, dd, J = 8.6 and 2.7Hz, CH=C(COCH₃)-CH) and 7.23 - 7.40 (5H, m, aromatics). m/e 234 (M⁺).

### Step B 2-Methoxy-5-hydroxymethyl-dibenzo[a,d] cycloheptene

To a solution of 2-methoxy-5-methylene-dibenzo [a,d]cycloheptene (12.8g) in dry THF (50ml), under an inert atmosphere, was added 9-borabicyclo[3,3,1]nonane (116ml of a 0.5 molar solution in THF) at ambient temperature, and the reaction mixture was then refluxed for 3h. After cooling in an ice bath the reaction was quenched by the slow addition of 2N sodium hydroxide solution (150ml) and 30% hydrogen peroxide (30ml). After stirring vigorously at 0°C for 45 minutes the reaction mixture was allowed to warm to room temperature over a period of 2h then extracted with diethyl ether (3x200ml). The combined organic layers were washed with brine (1x300ml), dried (Na₂SO₄), filtered and concentrated in vacuo to leave a residue, which was purified by chromatography on flash silica with 30% ethyl acetate in hexane as eluent, to give the title compound (10.9g) as an oil, δ (360MHz, CDCl₃) 3.77 - 3.81 (2H, d, J = 8.1Hz, CH₂), 3.80 (3H, s, OCH₃), 4.16 (1H, t, J = 8.1Hz, CH-CH₂OH), 6.79 (1H, d, J = 11.9Hz, Ar-CH_{A}=CH_{B}-Ar), 6.33 (1H, d, J = 2.6Hz, CH=C(OCH₃)-CH), 6.85 (1H, d, J = 11.9Hz, Ar-CH_{A}=CH_{B}-Ar), 6.89 (1H, dd, J = 8.3 and 2.6Hz, CH=C(OCH₃)CH) and 7.23 - 7.33 (5H, m, aromatics). m/e (CI⁺) 253 (M+1).

### Step C 2-Methoxy-5-p-toluenesulphonyloxymethyldibenzo[a,d]cycloheptene

To a solution of 2-methoxy-5-hydroxymethyldibenzo[a,d]cycloheptene (10.5g) in dry dichloromethane (150ml) was added p-toluenesulphonyl chloride (8.1g), pyridine (6.6g) and 4-dimethylamino pyridine (300mg). The reaction mixture was refluxed for 15h, cooled, washed with water (2x200ml), dried (Na₂SO₄), filtered and concentrated in vacuo. The residue was purified by flash silica chromatography using 15% ethyl acetate in hexane as eluent to give the title compound as an oil (13.5g). δ (360MHz, CDCl₃), 2.44 (3H, s, Ar-CH₃), 3.78 (3H, s, OCH₃), 4.19 - 4.31 (3H, m, CH-CH₂-OSO₂Ar), 6.58 (1H, d, J = 11.9Hz, Ar-CH=CH-Ar), 6.65 (1H, d, J = 11.9Hz, Ar-CH=CH-Ar), 6.68 (1H, d, J = 2.7, CH=C(OCH₃)-CH), 6.85 (1H, dd, J = 8.4 and 2.7Hz, CH=C(OCH₃)-CH) and 7.16 - 7.42 (9H, m, aromatics). m/e 406 (M⁺).

### Step D 2-Methoxy-5-hydroxy-dibenzo[a,e]cyclooctene and 2-Methoxy-6-hydroxy-dibenzo[a,e]cyclooctene

To a solution of 2-methoxy-5-p-toluenesulphonyloxymethyldibenzo[a,d]cycloheptene (13.5g) in glacial acetic acid (100ml) was added anhydrous sodium acetate (5.6g). The reaction mixture was heated under reflux for 15h then the solvent was removed under vacuum to leave a residue which was partitioned between dichloromethane (3x120ml) and water (120ml). The combined organic layers were dried (Na₂SO₄), filtered and concentrated in vacuo to leave a residue which was dissolved in 75% aqueous methanol (100ml) and treated with potassium hydroxide at 0°C until the pH was approximately 12. After 3h the reaction mixture was concentrated in vacuo and the presidue partitioned between water (100ml) and diethyl ether (3x100ml). The combined organic layers were dried (Na₂SO₄), filtered and concentrated in vacuo. Chromatography of the residue on flash silica with 10% ethyl acetate in hexane as eluent initially gave the less polar isomer, 2-methoxy-6-hydroxy-dibenzo [a,e]cyclooctene (1.6g), m.p. 97-99°C. δ (360MHz, CDCl₃) 3.25 (1H, dd, J = 13.8 and 10.0Hz, CH_{A}H_{B}-CH_{C}OH), 3.39 (1H, dd, J = 13.8 and 6.2Hz, CH_{A}H_{B}-CH_{C}OH), 3.72 (3H, s, OCH₃), 5.23 (1H, dd, J = 10.0 and 6.2Hz, CH_{A}H_{B}-CH_{C}-OH), 6.61 (1H, d, J = 2.7Hz, CH=C(OCH₃)-CH=CH), 6.70 (1H, dd, J = 8.4 and 2.7Hz, CH=C(OCH₃)-CH=CH), 6.77 (1H, d, J = 11.9, ArCH=CH-Ar), 6.87 (1H, d, J = 11.9Hz, Ar-CH=CH-Ar), 7.09 - 7.21 (3H, m, aromatics), 7.16 (1H, dd, J = 8.5 and 2.7Hz, CH=C(OCH₃)-CH=CH) and 7.44 (1H, dd, J = 8.6 and 2.7Hz, CH=C-CH₂OH). Irradiation of H_{B} (δ 3.39) gave a n.o.e to the proton at δ 7.16 which is on the same ring as the methoxy group and irradiation of H_{C} (δ 5.23) gave a n.o.e to the proton at δ 7.45 which is on the other aromatic ring. m/e 252 (M⁺). i.r. (νₘₐₓ nujol mull) 3400 - 3100 cm⁻¹ (br, OH). Found: C,78.8; H,6.5. C₁₇H₁₆O₂.O.35 H₂0 requires: C,78.9; H,6.5%. The more polar product next obtained, as an oil, was 2-methoxy-5-hydroxymethyldibenzo[a,d]cycloheptene (5.2g, 62%). 2-Methoxy-5-hydroxydibenzo[a,e]cyclooctene was not isolated.

### Step E 2-Methoxy-6-oxo-dibenzo[a,e]cyclooctene

To a solution of 2-methoxy-6-hydroxy-dibenzo[a,e] cyclooctene (1.5g) in dry dichloromethane (100ml) was added pyridinium dichromate (4.5g) and crushed 4A molecular sieves (2.3g). After stirring at room temperature for 2h, diethyl ether (200ml) was added and the reaction mixture was filtered through a plug of celite. The solvent was removed in vacuo and the residue purified by chromatography on flash silica with 5% ethyl acetate in hexane as eluent to give, as a colourless solid, the title compound (1.4g), m.p. 104-106°C. δ (360MHz, CDCl₃), 3.75 (3H, s, CH₃O), 3.99 (2H, s, CH₂-CO), 6.73 (1H, d, J = 2.7Hz, CH=(OCH₃)-CH), 6.86 (1H, dd, J = 8.5 and 2.7Hz, CH=C(OCH₃)-CH), 7.01 (2H, s, Ar-CH=CH-Ar), 7.29 - 7.51 (4H, m, aromatics) and 8.24 (1H, dd, J = 8.0 and 1.4Hz, CH=C-CO). m/e 250 (M⁺). i.r. (νₘₐₓ, nujol) 1665 cm⁻¹ (CO). Found: C,81.5; H,5.6. C₁₇H₁₄O₂ requires: C,81.0; H,5.8%

### Step F 2-Methoxy-13-hydroxy-6-methyl-5,6,11,12-tetrahydrodibenzo[a,e]cycloocten-6,11-imine

To a solution of 2-methoxy-6-oxo-dibenzo[a,e] cyclooctene (1.4g) in dry diethyl ether (60.0ml) at 0°C under an atmosphere of nitrogen was added methyl magnesium bromide (3.7ml of a 3 molar solution). After 45 minutes at 0°C the reaction mixture was quenched by the dropwise addition of saturated ammonium sulphate solution (25ml) and extracted into diethyl ether (3x50ml). The combined organic layers were dried (Na₂SO₄), filtered and evaporated to leave an oil (1.3g) which was dissolved in dry dichloromethane (10ml) and added to a solution containing dichloroacetic acid (7ml), sodium acetate (4.6g) and hydroxylamine hydrohloride (3.9g) in dichloromethane (37ml) which had been prepared 3h previously. The reaction mixture was stirred at room temperature for 8h. After cooling, 2N potassium hydroxide solution was added until the pH was 14 and the two phase mixture was stirred vigorously for 30 minutes. The organic layer was separated, washed with water (60ml) and brine (60ml), dried (Na₂SO₄), filtered and concentrated in vacuo. The crude residue was purified by chromatography on flash silica with 20% ethyl acetate in hexane to give, as a colourless solid, the title compound (0.8g), m.p. 185-197°C. The NMR spectrum showed a 2:1 mixture of atropisomers (360MHz, CDCl₃); major isomer: δ 1.62 (3H, s, CH₃), 2.37 (1H, d, J = 15.8Hz, CH_{A}H_{B}-C(CH₃), 2.55 (1H, dd, J = 16.0 and 5.2Hz, CH_{C}H_{D}-CH_{E} NOH), 4.04 (1H, d, J = 15.8, CH_{A}H_{B}-C(CH₃)), 4.2 (1H, d, J = 16.0Hz, CH_{C}H_{D}-CH_{E} NOH), 4.64 (1H, d, J = 5.2Hz, CH_{A}H_{B}-CH_{E} NOH) and 6.36 - 7.08 (7H, m, aromatics); minor isomer: δ 1.75 (3H, s, CH₃), 2.82 (1H, d, J = 15.5Hz, CH_{A}H_{B}-C(CH₃)), 3.03 (1H, dd, J = 16.0 and 7.2Hz, CH_{C}H_{D}-CH_{E}NOH), 3.36 (1H, d, J = 15.5Hz, CH_{A}H_{B}-C(CH₃)), 3.55 (1H, d, J = 16.0Hz, CH_{C}H_{D}-CH_{E} NOH), 4.73 (1H, d, J = 7.2Hz, CH_{C}H_{D}-CH_{E} NOH), 6.36 - 7.08 (7H, m, aromatics). m/e 281 (M⁺). Found: C,75.8; H,6.9; N,4.8. C₁₈H₁₉NO₂.0.25H₂O requires: C,75.6; H,6.9; N,4.9%.

### Step G 2-Methoxy-6-methyl-5,6,11,12-tetrahydrodibenzo [a,e]cycloocten-6,11-imine hydrochloride

To a solution of 2-methoxy-13-hydroxy-6-methyl-5,6,11,12-tetrahydrodibenzo[a,e]cycloocten-6,11-imine (0.8g) in glacial acetic acid (20ml) was added zinc dust (1.6g). The reaction mixture was heated at 65°C under an atmosphere of nitrogen. After 6h the mixture mixture was filtered and concentrated in vacuo to leave a residue which was partitioned between dichloromethane (50ml) and dilute sodium hydroxide solution (50ml). The organic layer was separated, dried (Na₂SO₄), filtered and evaporated to leave a residue which was dissolved in 5 molar hydrogen chloride in ethyl acetate (25ml). The solvent was evaporated to leave a solid which was recrystallised from methanol/ethyl acetate/diethyl ether. The title compound (290mg), m.p. 229-231°C, was collected by filtration and dried under high vacuum. δ (360MHz, DMSO) 1.87 (3H, s, CH₃), 3.04 (1H, d, J = 16.4Hz, CH_{A}H_{B}-C(CH₃)), 3.24 (1H, dd, J = 16.8 and 5.2Hz, CH_{C}H_{D}-CH_{E} NOH), 3.76 (1H, d, J = 16.4Hz, CH_{A}H_{B}-C(CH₃)), 5.15 (1H, d, J = 5.2Hz, CH_{C}H_{D}-CH_{E} NOH), 6.49 (2H, m, CH_{F}=C(OCH₃)-CH_{G}=CH_{H}), 6.79 (1H, d, J = 8.7Hz, CH_{F}=C(OCH₃)-CH_{G}=CH_{H}), 7.16 - 7.26 (4H, m, aromatics) and 10.22 - 10.40 (2H, m, NH₂). Irradiation of H_{H} (δ 6.79) gave a n.o.e to H_{A} (δ 3.04) and irradiation of the multiplet at δ 6.49 (H_{F} and H_{G}) gave a n.o.e to H_{C} (δ 3.24). m/e 265 (M⁺); Found: 265.1444; C₁₈H₁₉NO requires: 265.1466. Found: C,70.31; H,6.70; N,4.50. C₁₈H₁₉NO.HCl.H₂0 requires: C,70.37; H,6.75: N,4.56%.

### EXAMPLE 13

### 2-Hydroxy-6-methyl-5,6,11,12-tetrahydrodibenzo[a,e] cycloocten-6,11-imine hydrochloride

To a solution of 2-methoxy-6-methyl-5,6,11,12-tetrahydrodibenzo[a,e]cycloocten-6,11-imine (0.2g) in dichloromethane (50ml) at -78°C under an atmosphere of nitrogen was added, dropwise, boron tribromide (2.26ml). The reaction was stirred at -10°C for 2h and then chilled to -78°C whereupon methanol (5ml) was added dropwise. The mixture was basified with 2N sodium hydroxide then 2N hydrochloric acid was added to pH9. The mixture was extracted with dichloromethane (4x50ml) and the organic phases were combined, dried (Na₂SO₄), filtered and concentrated in vacuo to leave a residue which was purified by chromatography using 10% methanol and 1% ammonia in dichloromethane as eluent. The product was dissolved in 5 molar hydrogen chloride in ethyl acetate (15ml) and methanol (5ml). The solvent was evaporated to leave a solid which was recrystallised from methanol and ether. The title compound (0.12g), m.p. 268°C, was collected by filtration and dried under high vacuum. δ (360MHz, DMSO), 1.86 (3H, s, CH₃), 2.97 (1H, d, J = 16.4Hz, CH_{A}H_{B}-C(CH₃)), 3.14 (1H, dd, J = 16.7 and 5.2Hz, CH_{C}-H_{D}-CH_{E}NH), 3.64 (1H, d, J = 16.7Hz, CH_{C}H_{D}-CH_{E}NH), 3.73 (1H, d, J = CH_{A}H_{B}-C(CH₃)), 5.11 (1H, d, J = 5.2Hz, CH_{C}H_{D}-CH_{F} NH), 6.31 (2H, m, CH=C(OH)-CH=CH), 6.65 (1H, d, J = 7.8Hz, CH=C(OH)-CH=CH) and 7.15 - 7.27 (4H, m, aromatics). m/e found: 251.1315. C₁₇H₁₇NO requires: 251.1310. Found C,70.0; H,6.3; N,4.8. C₁₇H₁₇NO. HCl.O.25H₂O requires: C,69.9; H,6.4; N,4.8%.

### EXAMPLE 14

### 5,6,11,12-Tetrahydrodibenzo[a,e]cycloocten-6,11-imine hydrochloride

### Step A 5-Hydroxyimino-dibenzo[a,e]cyclooctene

To a solution of 5-oxo-dibenzo[a,e]cyclooctene (1g, Example 1, step E) in ethanol (40ml) was added hydroxylamine hydrochloride (0.316g) and pyridine (0.404ml). This mixture was heated to reflux for 8hr, cooled to room temperature and evaporated to dryness. To the residue was added ethyl acetate (100ml), water (100ml),then sodium hydroxide (1M) until pH 10. The aqueous layer was extracted with ethyl acetate (200ml), the combined organic layers were washed with water (100ml), brine (100ml) then dried and evaporated to yield the crude product. Recrystallisation from ethyl acetate-hexane gave pure 5-hydroxyimino-dibenzo[a,e]cyclooctene (0.72g, m.p. 191-92°C).

### Step B 13-Hydroxy-5,6,11,12-tetrahydrodibenzo-[a,e] cycloocten-6,11-imine

To a solution of the oxime (step A, 0.25g) in dry methanol was added methyl orange (3mg) and sodium cyanoborohydride (0.132g), followed by dropwise addition of a solution of concentrated hydrochloric acid (250»l) in methanol (3ml), with the pH being maintained between 3-4. After the addition was complete, the mixture was stirred for 2 hours. This addition procedure was repeated three times using a total of 0.53g sodium cyanoborohydride and 1ml concentrated hydrochloric acid in methanol (12ml). After stirring overnight, ethyl acetate (50ml) and water (50ml) were added, followed by sodium hydroxide (1M) to give pH 14. The aqueous layer was extracted with ethyl acetate (150ml) and the combined organic layers washed with water (100ml), brine (100ml) and then dried and evaporated to yiled a mixture of 5-hydroxyl-aminodibenzo[a,e]cyclooctene and 13-hydroxy-5,6,11,12-tetrahydrodibenzo[a,e]cycloocten-6 ,11-imine. This mixture was heated in xylene (10ml) at 130°C for 10 mins, cooled to room temperature and evaporated to yield a crude product which was purified by chromatography (ethyl acetate hexane) to give pure 13-hydroxy-5,6,11,12-tetrahydrodibenzo[a,e] cycloocten-6,11-imine (0.165g, m.p. 175-79°C).

### Step C 5,6,11,12-Tetrahydrodibenzo[a,e]cycloocten-6, 11-imine hydrochloride

To a solution of the hydroxy imine (step B, 0.165g) in glacial acetic acid (5ml) was added zinc powder (0.165g) and the suspension heated to 60°C for 8 hours. The mixture was cooled to room temperature and filtered. Sodium hydroxide (1M) was added to the filtrate until pH 12 and the solution was extracted into ethyl acetate (150ml). The combined organic layers were washed with water (100ml), brine (100ml) and then dried and evaporated. The residue was purified using chromatography (methanol-dichloromethane) and treated with saturated hydrogen chloride in ethyl acetate (1ml) to yield pure 5,6,11,12-tetrahydrodibenzo[a,e]cycloocten-6,11-imine hydrochloride (45mg, mp >290°C), δ (DMSO-d₆, 360MHz) 3.25 (2H, dd, J = 16.8 and 5.5Hz, CH_{A}H_{B}), 3.82 (2H, d, J = 16.8Hz, CH_{A}H_{B}), 5.22 (2H, d, J = 5.5Hz, CH_{C}), 6.92 (4H, s, aromatics), 7.13 - 7.17 (2H, m, aromatics), 7.24 - 7.28 (2H, m, aromatics), and 10.17 (2H, bs, NH₂). N.o.e experiments confirmed the structure.

### EXAMPLE 15

### 11-Hydroxy-6-methyl-5,6,11,12-tetrahydrodibenzo[a,e ]cycloocten-6,11-imine hydrochloride

### Step A 11-Acetoxy-13-hydroxy-6-methyl-5,6,11,12-tetrahydrodibenzo[a,e]cycloocten-6,11-imine

To a solution of 13-hydroxy-6-methyl-5,6,11,12-tetrahydrodibenzo[a,e]cycloocten-6,11-imine (0.2g; Example 1, step G) in glacial acetic acid (15ml) at room temperature was added potassium bromide (20mg) followed by manganese (III) acetate dihydrate (0.256g). The mixture was stirred for one hour, ethyl acetate (50ml) was added, then sodium hydroxide (2M) was added until pH 12. The two layers were separated and the aqueous layer extracted with ethyl acetate (150ml), the combined organic layers washed with water (100ml), brine (100ml), then dried and evaporated. The residue was purified using chromatography (ethyl acetate-hexane) to yield the desired acetate (0.148g), mp 135-38°C (dec).

### Step B 11-Hydroxy-6-methyl-5,6,11,12-tetrahydrodibenzo[a,e]cycloocten-6,11-imine hydrochloride

To a solution of the hydroxy imine (step A, 0.14g) in glacial acetic acid (5ml) was added zinc powder (0.14g) and the suspension stirred at 60°C for 12 hours, then cooled to room temperature and filtered. To the filtrate was added sodium hydroxide (2M) until pH 12 and the solution was extracted with ethyl acetate (150ml), the combined organic layers washed with water (100ml), brine (100ml) then dried and evaporated. Purification of the residue by chromatography (methanol-dichloromethane), followed by treatment with saturated hydrogen chloride in ethyl acetate (2ml) gave pure 11-hydroxy-6-methyl-5,6,11,12-tetrahydrodibenzo[a,e]cycloocten-6,11-imine hydrochloride (45mg, mp >280°C). δ (DMSO-d₆, 360MHz) 1.88 (3H, s, CH₃), 3.05 (1H, d, J = 16.4Hz, CH₃C-CH_{A}H_{B}), 3.27 (1H, d, J = 16.0Hz, CH₃C-CH_{A}CH_{B}), 3.96 (1H, d, J = 16.4Hz, HO-C-CH_{C}H_{D}), 4.18 (1H, d, J = 16.0Hz, HO-C-CH_{C}H_{D}), 6.85 - 6.94 (4H, m, aromatics), 7.19 - 7.25 (4H, m, aromatics), 8.39 (1H, bs, OH) and 9.17 and 11.00 (2H, bs, NH₂).

### EXAMPLE 16

### 6,11-Dimethyl-5,6,11,12-tetrahydrodibenzo[a,e]cycloocten-6,11-imine hydrochloride

### Step A 6-Methyl-13-p-toluenesulphonyl-5,6,11,12-tetrahydrodibenzo[a,e]cycloocten-6,11-imine

To a solution of 6-methyl-5,6,11,12-tetrahydrodibenzo[a,e]cycloocten-6,11-imine (Example 1, 2.91g, 12.4mmol) in dichloromethane (100ml) was added p-toluenesulphonyl chloride (4.72g, 24.8mmol) followed by 4-dimethylaminopyridine (3.03g, 24.8mmol) and the resulting mixture heated at reflux under nitrogen for 14h. The crude reaction mixture was purified by flash chromatography using 50% dichloromethane in petroleum ether (b.pt. 60-80°C) as eluent to give the crude product which was triturated with pentane to give the title compound (3.85g) m.p. 165-167°C. δ (250MHz, CDCl₃) 1.74 (3H, s, CH₃), 2.35 (3H, s, Ar-CH₃), 2.88 (1H, d, J = 16.0Hz, CH_{A}H_{B}), 3.18 (1H, dd, J = 4.40 and 16.3Hz, CH_{C}H_{D}-CH_{E}), 4.06 (1H, br d, J = 16.1Hz, CH_{C}H_{D}-CH_{E}), 4.16 (1H, d, J = 15.9Hz, CH_{A}H_{B}) 5.51 (1H, dd, J = 2.14 and 4.20Hz, CH_{C}H_{D}-CH_{E}), 6.80 - 6.92 (5H, m, aromatics), 7.03 - 7.06 (3H, m, aromatics), 7.18 (2H, d, J = 7.95Hz, aromatics) and 7.68 (2H, d, J = 8.35Hz, aromatics). m/e (EI) 234 (M-CH₃C₆H₄SO₂)⁺; (CI⁺) 390 (M+1)⁺.

### Step B 6,11-Dimethyl-5,6,11,12-tetrahydrodibenzo[a,e] cycloocten-6,11-imine hydrochloride

To a solution of 6-methyl-13-p-toluenesulphonyl-5, 6,11,12-tetrahydrodibenzo[a,e]cycloocten-6,11-imine (500mg) in anhydrous tetrahydrofuran (25ml) at room temperature and under an atmosphere of nitrogen was added methyllithium (1.7ml of a 1.6M solution in diethyl ether, 2 molar equivalents) and the resulting mixture was stirred for 7 minutes. The reaction was then quenched with water, diluted with saturated brine and extracted twice with diethyl ether. The combined ethereal extracts were dried over anhydrous sodium sulphate and evaporated. This procedure was then repeated in four separate batches (using 3x900mg and 1x500mg of 6-methyl-13-p-toluenesulphonyl-5,6,11,12-tetrahydrodibenzo[a,e]cycloocten-6,11-imine) and all the resulting crude products were combined to give an oil (2.63g). This was purified by flash chromatography using 2% methanol/0.1% ammonia in dichloromethane as the eluent to give the crude product (355mg). This was further purified by Lobar chromatography using 4% methanol-0.2% ammonia in dichloromethane as the eluent to give 280mg of the free base. A portion of this (150mg) was dissolved in ethyl acetate, filtered and treated with excess hydrogen chloride in diethyl ether which, after evaporation, yielded a product which was recrystallised from methanol/ethyl acetate/diethyl ether to give the title compound as colourless crystals, m.p. 230-234°C (sub). δ (250MHz, CDCl₃) 2.23 (6H, s, 2xCH₃), 2.90 (2H, d, J = 16.4Hz, 2xCH_{A}H_{B}), 4.36 (2H, d, J = 16.3Hz, 2xCH_{A}H_{B}), 6.76 - 6.79 (2H, m, aromatics), 7.86 - 6.92 (2H, m, aromatics), 6.97 - 7.02 (2H, m, aromatics) and 7.09 - 7.14 (2H, m, aromatics). MS: m/e (EI) 249 (M-HCl)⁺, 145; (CI⁺) 250 (M-HCl +1)⁺, 145; (CI⁻)248 (M-HCl-1)⁺, 127. Found: C,75.01; H,7.05; N,4.90. C₁₈H₂₀ClN.0.15H₂O requires: C,74.93; H,7.09; N,4.85%.

### EXAMPLE 17

### 6-Ethyl-5,6,11,12-tetrahydrodibenzo[a,e]cycloocten-6,11-imine hydrochloride

### Step A 5-Hydroxy-5-ethyl-dibenzo[a,e]cyclooctene

To a stirred solution of 5-oxo-dibenzo[a,e]cyclooctene (1.1g) in anhydrous ether (50ml) at 0°C under nitrogen, was added over 10 minutes, a 2.0M solution of ethyl magnesium bromide in tetrahydrofuran (2.5ml). The mixture was stirred for 30 minutes, then water (20ml) was added, the solvents were evaporated and the residue extracted with ether, the organic layer washed with water, dried and evaporated to dryness to give the pure alcohol as an oil which crystallized slowly on standing (710mg), m.p. 81-5°C.

### Step B 6-Ethyl-13-hydroxy-5,6,11,12-tetrahydrodibenzo [a,e]cycloocten-6,11-imine

To a suspension of anhydrous sodium acetate (1.15g) and hydroxylamine hydrochloride (0.97g) and the preceding alcohol (350mg) in dichloromethane at reflux was added dichloroacetic acid (2.5ml) in dichloromethane (15mg) over 20 minutes with vigorous stirring. The mixture was stirred at reflux for 30 minutes, then cooled and 2M aqueous sodium hydroxide added to pH 12. The organic layer was separated, washed with water and brine, than dried and evaporated. Crystallisation of the residue from n-hexane gave the desired cyclised hydroxylamine (150mg), m.p. 128-31°.

### Step C 6-Ethyl-5,6,11,12-tetrahydrodibenzo[a,e] cycloocten-6,11-imine hydrochloride

To a solution of the N-hydroxy compound from Step B (65mg) in glacial acetic acid (2ml) was added zinc dust (250mg) and the mixture was heated at 65°C for 90 minutes, then filtered, evaporated to dryness and portitioned between ether (10ml) and 1M aqueous sodium hydroxide (20ml). The ether layer was washed with water, dried and evaporated and the residue chromatographed on flash silica with 3% methanol/chloroform as eluent. The combined fractions were evaporated to dryness, then dissolved in ethyl acetate and treated with hydrogen chloride in ethyl acetate to give the hydrochloride salt as a buff solid (38.2g), m.p. 240-5°C. δ (360MHz, CDCl₃) 1.47 (H, t, J = 7.5Hz, CH₃), 2.47 (2H, q, J = 7.5Hz, CH₂CH₃), 2.88 (1H, d, J = 16.2Hz, C(Et)CH_{A}H_{B}), 3.17 (1H, dd, J = 16.6Hz and 5.9Hz, CHCH_{A}H_{B}), 4.28 (1H, d, J = 16.2Hz, C(Et)CH_{A}H_{B}) 4.35 (1H, d, J = 16.6Hz, CHCH_{A}H_{B}), 5.41 (1H, t, J = 5.9Hz, CH), 6.80-7.12, (8H, m, aromatics) 10.4 (1H, brs, NH⁺) and 10.9 (1H, brs, NH⁺).

### EXAMPLE 18

### 6-Methyl-5,6,6a,7,8,9,10,10a,11,12-decahydro[a,e] cyclooctene-6,11-imine hydrochloride

A solution of 6-methyl-5,6,11,12-tetrahydrodibenzo [a,e]cycloocten-6,11-imine hydrochloride (100mg) in glacial acetic acid (5ml) was hydrogenated for 12h at 50psi in the presence of platinum oxide catalyst (40mg). The catalyst was removed by filtration and the solvents evaporated to give the crude product, which was partitioned between 2M aqueous sodium hydroxide (20ml) and ether (30ml). The organic extract was washed, dried and evaporated to give the product as the free base. Treatment of this with hydrogen chloride in ethyl acetate followed by trituration with ether, gave the required hydrochloride as a white solid (90mg), m.p. 230-3°C.

### EXAMPLE 19

### 6,13-Dimethyl-5,6,11,12-tetrahydrodibenzo[a,e] cycloocten-6,11-imine hydrochloride

To a solution of 6-methyl-5,6,11,12-tetrahydrodibenzo[a,e]cycloocten-6,11-imine (0.50g) in dichloromethane (80ml) at 0°C was added methyl iodide (one mole equivalent, 0.132ml). The solution was stirred at room temperature overnight, then a further five mole equivalents of methyl iodide was added. After 4h, the mixture was treated with concentrated ammonia (5ml), then washed with sodium hydroxide solution, the organic layer removed, dried and evaporated. Crystallisation of the residue gave the N,N-dimethyl quaternary salt (90mg), m.p. 259-261°C. Found: C,58.22; H,5.59; N,3.57 C₁₉H₂₂IN requires C,58.32; H,5.67; N,3.58%). The mother liquors were evaporated and chromatographed on silica gel, eluting with dichloromethane containing 4.5% methanol and 0.5% ammonia to give the N-methyl compound as the free base. Addition of a saturated solution of hydrogen chloride in ethyl acetate to a methanol solution of the free base, followed by evaporation and recrystallisation of the residue from ether-dichloromethane gave the title compound (23mg), m.p. 272-273°C. (Found: C,71.14; H,6.64; N,4.56. C₁₈H₁₉N.1.5HCl requires: C,71.11; H,6.80; N,4.60%). m/e : found 249.15080; C₁₈H₁₉N requires 240.15175.

### EXAMPLE 20

### Tablet Preparation

Tablets containing 1.0, 2.0, 25.0, 26.0, 50.0 and 100.0mg, respectively, of the following compounds are prepared as illustrated below:
6-methyl-5,6,11,12-tetrahydrodibenzo[a,e]cycloocten-6, 11-imine
3-bromo-6-methyl-5,6,11,12-tetrahydrodibenzo[a,e] cycloocten-6,11-imine
11-hydroxy-6-methyl-5,6,11,12-tetrahydrodibenzo[a,e] cycloocten-6,11-imine
6,11-dimethyl-5,6,11,12-tetrahydrodibenzo[a,e] cycloocten-6,11-imine

| TABLE FOR DOSES CONTAINING FROM 1-25 MG OF THE ACTIVE COMPOUND | | | |
|---|---|---|---|
| | Amount-mg | | |
| Active Compound | 1.0 | 2.0 | 25.0 |
| Microcrystalline cellulose | 49.25 | 48.75 | 37.25 |
| Modified food corn starch | 49.25 | 48.75 | 37.25 |
| Maganesium stearate | 0.50 | 0.50 | 0.50 |
| Active Compound | 26.0 | 50.0 | 100.0 |
| Microcrystalline cellulose | 52.0 | 100.0 | 200.0 |
| Midified food corn starch | 2.21 | 4.25 | 8.5 |
| Maganesium stearate | 0.39 | 0.75 | 1.5 |

All of the active compound, lactose, and a portion of the corn starch are mixed and granulated to a 10% corn starch paste. The resulting granulation is sieved, dried and blended with the remainder of the corn starch and the magnesium stearate. The resulting granulation is then compressed into tablets containing 1.0 mg, 2.9 mg, 25.0 mg, 26.0 mg, 50.0 mg and 100.0 mg of active ingredient per tablet.

## Claims (Claims for the following Contracting State(s): AT, BE, CH, DE, FR, GB, IT, LI, LU, NL, SE)

1. A compound of formula I: 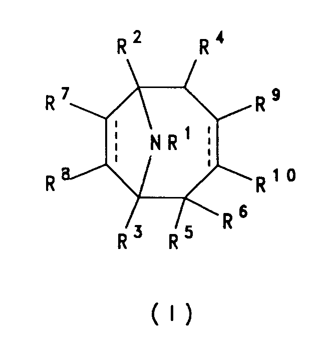 or a salt thereof, wherein:
the dotted lines represent optional double bonds;
R¹ represents hydrogen, hydroxy, C₂₋₆ alkenyl, alkyl containing from 1 to 6 carbon atoms, aminoalkyl containing from 1 to 6 carbon atoms or hydroxyalkyl containing from 1 to 6 carbon atoms;
R², R³, R⁴, R⁵ and R⁶ independently represent hydrogen, hydroxy, fluoro, C₂₋₆ alkenyl, aryl, alkyl containing from 1 to 6 carbon atoms, or alkyl containing from 1 to 6 carbon atoms substituted with aryl, amino, hydroxy, carboxy or fluoro; and
R⁷, R⁸, R⁹ and R¹⁰ independently represent hydrogen, hydrocarbon having up to 18 carbon atoms, or a heterocyclic group, which hydrocarbon or heterocyclic groups are optionally substituted with a group selected from halogen, C₁₋₆ alkyl, aryl, arylalkyl, C₁₋₆ alkoxy, halo(C₁₋₆)alkyl, hydroxy, amino, nitro, carboxy, C₁₋₆ alkoxycarbonyl, C₁₋₆ alkoxycarbonyl-(C₁₋₆)alkyl, C₁₋₆ alkylcarbonyloxy and C₁₋₆ alkylcarbonyl groups provided that R⁷, R⁸, R⁹ and R¹⁰ are not simultaneously hydrogen; or
R⁷ and R⁸ and/or R⁹ and R¹⁰ may complete a saturated or unsaturated C₄₋₉ hydrocarbon or heterocyclic ring.

2. A compound as claimed in claim 1 wherein R² and R⁶ independently represent hydrogen, hydroxy or C₁₋₄ alkyl.

3. A compound of formula II: 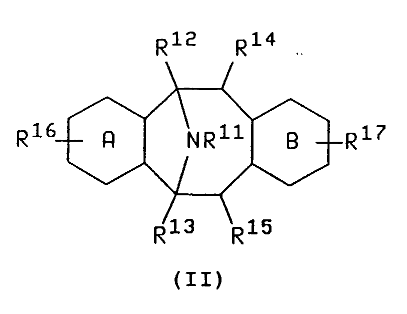 or a salt thereof, wherein rings A and B independently represent cyclohexane, cyclohexene, cyclohexadiene or benzene rings; R¹¹ is as defined in claim 1 for R¹; and R¹² to R¹⁷ independently represent hydrogen, hydroxy, C₁₋₆ alkyl, aryl, arylalkyl, C₁₋₆ alkoxy or halogen.

4. A compound as claimed in claim 3 wherein rings A and B represent benzene rings and R¹⁶ and R¹⁷ independently represent hydrogen, methyl, hydroxy, methoxy, bromo or chloro.

5. A compound as claimed in claim 3 or claim 4 wherein R¹³ represents methyl.

6. A compound as claimed in claim 1 selected from:
6-methyl-5,6,11,12-tetrahydrodibenzo[a,e]cycloocten-6,11-imine;
11-n-butyl-6-methyl-5,6,11,12-tetrahydrodibenzo[a,e]cycloocten-6,11-imine;
5,6-dimethyl-5,6,11,12-tetrahydrodibenzo[a,e]cycloocten-6,11-imine;
2-bromo-6-methyl-5,6,11,12-tetrahydrodibenzo[a,e]cycloocten-6,11-imine;
9-bromo-6-methyl-5,6,11,12-tetrahydrodibenzo[a,e]cycloocten-6,11-imine;
3,6-dimethyl-5,6,11,12-tetrahydrodibenzo[a,e]cycloocten-6,11-imine;
6,8-dimethyl-5,6,11,12-tetrahydrodibenzo[a,e]cycloocten-6,11-imine;
2-chloro-6-methyl-5,6,11,12-tetrahydrodibenzo[a,e]cycloocten-6,11-imine;
9-chloro-6-methyl-5,6,11,12-tetrahydrodibenzo[a,e]cycloocten-6,11-imine;
3-bromo-6-methyl-5,6,11,12-tetrahydrodibenzo[a,e]cycloocten-6,11-imine;
6-methyl-5,6,6a-exo,7,8,9,10,10a-exo,11,12-decahydrodibenzo[a,e]cycloocten-6,11-imine;
6-methyl-5,6,6a-endo,7,8,9,10,10a-endo,11,12-decahydrodibenzo[a,e]cycloocten-6,11-imine;
5,6,11,12-tetrahydrodibenzo[a,e]cycloocten-6,11-imine;
6,11-dimethyl-5,6,11,12-tetrahydrodibenzo[a,e]cycloocten-6,11-imine;
2-methoxy-6-methyl-5,6,11,12-tetrahydrodibenzo[a,e]cycloocten-6,11-imine;
3-methoxy-6-methyl-5,6,11,12-tetrahydrodibenzo[a,e]cycloocten-6,11-imine;
2-hydroxy-6-methyl-5,6,11,12-tetrahydrodibenzo[a,e]cycloocten-6,11-imine;
3-hydroxy-6-methyl-5,6,11,12-tetrahydrodibenzo[a,e]cycloocten-6,11-imine;
12-exo-hydroxy-6-methyl-5,6,11,12-tetrahydrodibenzo[a,e]cycloocten-6,11-imine;
1-chloro-6-methyl-5,6,11,12-tetrahydrodibenzo[a,e]cycloocten-6,11-imine;
10-chloro-6-methyl-5,6,11,12-tetrahydrodibenzo[a,e]cycloocten-6,11-imine;
6-ethyl-5,6,11,12-tetrahydrodibenzo[a,e]cycloocten-6,11-imine;
8-bromo-6-methyl-5,6,11,12-tetrahydrodibenzo[a,e]cycloocten-6,11-imine;
11-hydroxy-6-methyl-5,6,11,12-tetrahydrodibenzo[a,e]cycloocten-6,11-imine;
6,13-dimethyl-5,6,11,12-tetrahydrodibenzo[a,e]cycloocten-6,11-imine;
and salts thereof.

7. A pharmaceutical composition comprising a compound as claimed in any one of the preceding claims in association with a pharmaceutically acceptable carrier or excipient.

8. A compound as claimed in any one of claims 1 to 6 for use as a therapeutic agent.

9. The use of a compound as claimed in any one of claims 1 to 6 for the preparation of a medicament for the treatment and/or prevention of convulsions and/or of neurodegenerative disorders.

10. A process for the preparation of a compound as claimed in any one of claims 1 to 6 which comprises:
(A) for the preparation of a compound of formula I wherein R¹ is other than hydroxy:
reduction of a compound of formula III: 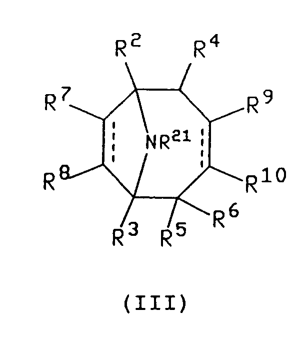 wherein R²¹ represents hydroxy or C₁₋₆ alkoxy, and R² to R¹⁰ are as defined in claim 1; and, when R¹ is other than hydrogen, alkylation or alkenylation of the product; or
(B) for the preparation of a compound of formula I wherein R² represents alkyl, carboxymethyl or halogen:
reaction of a compound of formula VI: 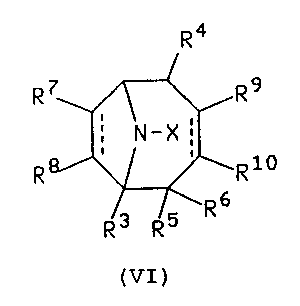 wherein R³ to R¹⁰ are as defined in claim 1 and X represents a leaving group; with an anion ⁻R²; or
(C) for the preparation of a compound of formula I wherein R² and/or R³ represent hydroxy:
treatment of a compound of formula III as defined above, in which R² and/or R³ is hydrogen, with manganese acetate; reduction of the product thereby obtained; and, when R¹ is other than hydrogen, alkylation or alkenylation of the resulting product; or
(D) for the preparation of a compound of formula I wherein R⁴ and/or R⁵ represent hydroxy when R⁶ represents hydrogen:
reduction of the corresponding oxo or dioxo compound, in which the nitrogen atom is protected; deprotection of the nitrogen atom; and, if desired, separation of the product thereby obtained into its individual endo and exo isomers by conventional techniques; or
(E) for the preparation of a compound of formula I wherein R², R³, R⁴ and/or R⁵ represent fluorine:
treatment of the corresponding hydroxy compound with diethylaminosulphur trifluoride; or
(F) for the preparation of a compound of formula VII: 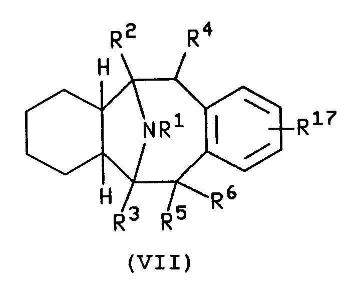 wherein R¹ and R⁶ are as defined in claim 1 and R¹⁷ is as defined in claim 3:
reduction of the corresponding dibenzo compound.

## Claims (Claims for the following Contracting State(s): ES)

1. A process for the preparation of a compound of formula I: 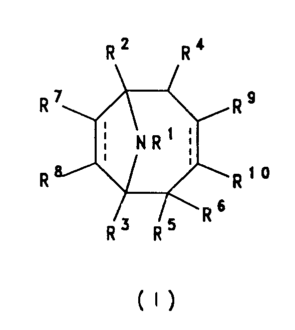 or a salt thereof, wherein:
the dotted lines represent optional double bonds;
R¹ represents hydrogen, hydroxy, C₂₋₆ alkenyl, alkyl containing from 1 to 6 carbon atoms, aminoalkyl containing from 1 to 6 carbon atoms or hydroxyalkyl containing from 1 to 6 carbon atoms;
R², R³, R⁴, R⁵ and R⁶ independently represent hydrogen, hydroxy, fluoro, C₂₋₆ alkenyl, aryl, alkyl containing from 1 to 6 carbon atoms, or alkyl containing from 1 to 6 carbon atoms substituted with aryl, amino, hydroxy, carboxy or fluoro; and
R⁷, R⁸, R⁹ and R¹⁰ independently represent hydrogen, hydrocarbon having up to 18 carbon atoms, or a heterocyclic group, which hydrocarbon or heterocyclic groups are optionally substituted with a group selected from halogen, C₁₋₆ alkyl, aryl, arylalkyl, C₁₋₆ alkoxy, halo(C₁₋₆)alkyl, hydroxy, amino, nitro, carboxy, C₁₋₆ alkoxycarbonyl, C₁₋₆ alkoxycarbonyl(C₁₋₆)alkyl, C₁₋₆ alkylcarbonyloxy and C₁₋₆ alkylcarbonyl groups provided that R⁷, R⁸, R⁹ and R¹⁰ are not simultaneously hydrogen; or
R⁷ and R⁸ and/or R⁹ and R¹⁰ may complete a saturated or unsaturated C₄₋₉ hydrocarbon or heterocyclic ring; which process comprises:
(A) for the preparation of a compound of formula I wherein R¹ is other than hydroxy:
reduction of a compound of formula III: 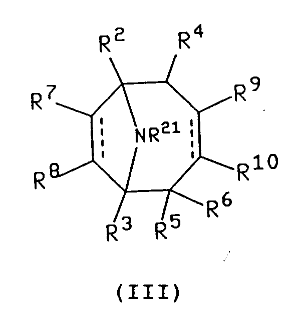 wherein R²¹ represents hydroxy or C₁₋₆ alkoxy, and R² to R¹⁰ are as defined above; and, when R¹ is other than hydrogen, alkylation or alkenylation of the product; or
(B) for the preparation of a compound of formula I wherein R² represents alkyl, carboxymethyl or halogen: reaction of a compound of formula VI: 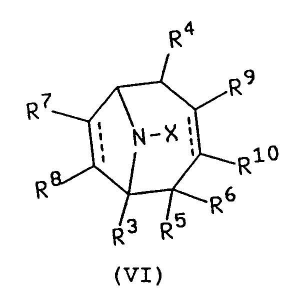 wherein R³ to R¹⁰ are as defined above and X represents a leaving group; with an anion ⁻R²; or
(C) for the preparation of a compound of formula I wherein R² and/or R³ represent hydroxy:
treatment of a compound of formula III as defined above, in which R² and/or R³ is hydrogen, with manganese acetate; reduction of the product thereby obtained; and, when R¹ is other than hydrogen, alkylation or alkenylation of the resulting product; or
(D) for the preparation of a compound of formula I wherein R⁴ and/or R⁵ represent hydroxy when R⁶ represents hydrogen:
reduction of the corresponding oxo or dioxo compound, in which the nitrogen atom is protected; deprotection of the nitrogen atom; and, if desired, separation of the product thereby obtained into its individual endo and exo isomers by conventional techniques; or
(E) for the preparation of a compound of formula I wherein R², R³, R⁴ and/or R⁵ represent fluorine:
treatment of the corresponding hydroxy compound with diethylaminosulphur trifluoride; or
(F) for the preparation of a compound of formula VII: 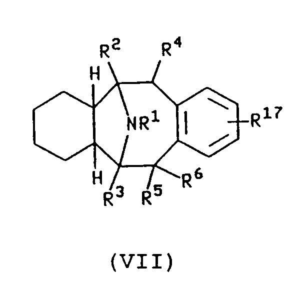 wherein R¹ and R⁶ are as defined in claim 1 and R¹⁷ represents hydrogen, hydroxy, C₁₋₆ alkyl, aryl, arylalkyl, C₁₋₆ alkoxy or halogen:
reduction of the corresponding dibenzo compound.

2. A process as claimed in claim 1 for the preparation of a compound wherein R² and R⁶ independently represent hydrogen, hydroxy or C₁₋₄ alkyl.

3. A process as claimed in claim 1 for the preparation of a compound of formula II: 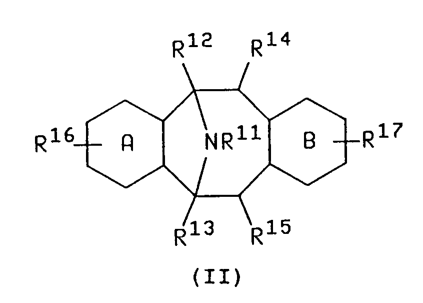 or a salt thereof, wherein rings A and B independently represent cyclohexane, cyclohexene, cyclohexadiene or benzene rings; R¹¹ is as defined in claim 1 for R¹; and R¹² to R¹⁷ independently represent hydrogen, hydroxy, C₁₋₆ alkyl, aryl, arylalkyl, C₁₋₆ alkoxy or halogen.

4. A process as claimed in claim 3 for the preparation of a compound wherein rings A and B represent benzene rings and R¹⁶ and R¹⁷ independently represent hydrogen, methyl, hydroxy, methoxy, bromo or chloro.

5. A process as claimed in claim 3 or claim 4 for the preparation of a compound wherein R¹³ represents methyl.

6. A process as claimed in claim 1 for the preparation of a compound selected from:
6-methyl-5,6,11,12-tetrahydrodibenzo[a,e]cycloocten-6,11-imine;
11-n-butyl-6-methyl-5,6,11,12-tetrahydrodibenzo[a,e]cycloocten-6,11-imine;
5,6-dimethyl-5,6,11,12-tetrahydrodibenzo[a,e]cycloocten-6,11-imine;
2-bromo-6-methyl-5,6,11,12-tetrahydrodibenzo[a,e]cycloocten-6,11-imine;
9-bromo-6-methyl-5,6,11,12-tetrahydrodibenzo[a,e]cycloocten-6,11-imine;
3,6-dimethyl-5,6,11,12-tetrahydrodibenzo[a,e]cycloocten-6,11-imine;
6,8-dimethyl-5,6,11,12-tetrahydrodibenzo[a,e]cycloocten-6,11-imine;
2-chloro-6-methyl-5,6,11,12-tetrahydrodibenzo[a,e]cycloocten-6,11-imine;
9-chloro-6-methyl-5,6,11,12-tetrahydrodibenzo[a,e]cycloocten-6,11-imine;
3-bromo-6-methyl-5,6,11,12-tetrahydrodibenzo[a,e]cycloocten-6,11-imine;
6-methyl-5,6,6a-exo,7,8,9,10,10a-exo,11,12-decahydrodibenzo[a,e]cycloocten-6,11-imine;
6-methyl-5,6,6a-endo,7,8,9,10,10a-endo,11,12-decahydrodibenzo[a,e]cycloocten-6,11-imine;
5,6,11,12-tetrahydrodibenzo[a,e]cycloocten-6,11-imine;
6,11-dimethyl-5,6,11,12-tetrahydrodibenzo[a,e]cycloocten-6,11-imine;
2-methoxy-6-methyl-5,6,11,12-tetrahydrodibenzo[a,e]cycloocten-6,11-imine;
3-methoxy-6-methyl-5,6,11,12-tetrahydrodibenzo[a,e]cycloocten-6,11-imine;
2-hydroxy-6-methyl-5,6,11,12-tetrahydrodibenzo[a,e]cycloocten-6,11-imine;
3-hydroxy-6-methyl-5,6,11,12-tetrahydrodibenzo[a,e]cycloocten-6,11-imine;
12-exo-hydroxy-6-methyl-5,6,11,12-tetrahydrodibenzo[a,e]cycloocten-6,11-imine;
1-chloro-6-methyl-5,6,11,12-tetrahydrodibenzo[a,e]cycloocten-6,11-imine;
10-chloro-6-methyl-5,6,11,12-tetrahydrodibenzo[a,e]cycloocten-6,11-imine;
6-ethyl-5,6,11,12-tetrahydrodibenzo[a,e]cycloocten-6,11-imine;
8-bromo-6-methyl-5,6,11,12-tetrahydrodibenzo[a,e]cycloocten-6,11-imine;
11-hydroxy-6-methyl-5,6,11,12-tetrahydrodibenzo[a,e]cycloocten-6,11-imine;
6,13-dimethyl-5,6,11,12-tetrahydrodibenzo[a,e]cycloocten-6,11-imine;
and salts thereof.

7. A process for the preparation of a pharmaceutical composition which comprises mixing a compound prepared as claimed in any one of the preceding claims with a pharmaceutically acceptable carrier or excipient.

8. The use of a compound prepared as claimed in any one of claims 1 to 6 for the preparation of a medicament for the treatment and/or prevention of convulsions and/or of neurodegenerative disorders.

## Patentansprüche (Patentansprüche für folgende(n) Vertragsstaat(en): AT, BE, CH, DE, FR, GB, IT, LI, LU, NL, SE)

1. Eine Verbindung der Formel I 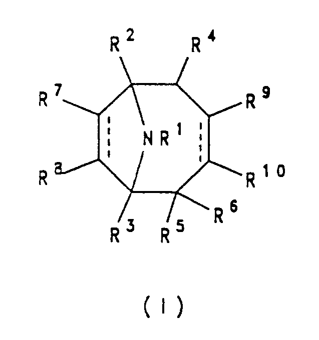 oder ein Salz hiervon, worin
die gestrichelten Linien fakultative Doppelbindungen bedeuten;
R¹ Wasserstoff, Hydroxy, C₂₋₆ Alkenyl, Alkyl, enthaltend 1 bis 6 Kohlenstoffatome, Aminoalkyl,enthaltend 1 bis 6 Kohlenstoffatome,oder Hydroxyalkyl,enthaltend 1 bis 6 Kohlenstoffatome, bedeutet;
R², R³, R⁴, R⁵ und R⁶ unabhängig voneinander Wasserstoff, Hydroxy, Fluor, C₂₋₆ Alkenyl, Aryl, Alkyl, enthaltend 1 bis 6 Kohlenstoffatome, oder Alkyl, enthaltend 1 bis 6 Kohlenstoffatome, substitutiert durch Aryl, Amino, Hydroxy, Carboxy oder Fluor, bedeuten; und
R⁷, R⁸, R⁹ und R¹⁰ unabhängig voneinander Wasserstoff, eine Kohlenwasserstoffgruppe mit bis zu 18 Kohlenstoffatomen oder eine heterocyclische Gruppe bedeuten, wobei die Kohlenwasserstoffgruppen oder die heterocyclischen Gruppen gegebenenfalls durch eine Gruppe substituiert sind, die aus Halogen, C₁₋₆ Alkyl, Aryl, Arylalkyl, C₁₋₆ Alkoxy, Halogen-(C₁₋₆)alkyl, Hydroxy, Amino, Nitro, Carboxy, C₁₋₆ Alkoxycarbonyl, C₁₋₆ Alkoxycarbonyl-(C₁₋₆)alkyl, C₁₋₆ Alkylcarbonyloxy und C₁₋₆ Alkylcarbonylgruppen ausgewählt ist, unter der Bedingung, daß R⁷, R⁸, R⁹ und R¹⁰ nicht gleichzeitig Wasserstoff sind; oder
R⁷ und R⁸ und/oder R⁹ und R¹⁰ einen gesättigten oder ungesättigten C₄₋₉ Kohlenwasserstoffring oder heterocyclischen Ring komplettieren können.

2. Eine Verbindung, wie sie im Anspruch 1 beansprucht ist, worin R² und R⁶ unabhängig voneinander Wasserstoff, Hydroxy oder C₁₋₄ Alkyl bedeuten.

3. Eine Verbindung der Formel II 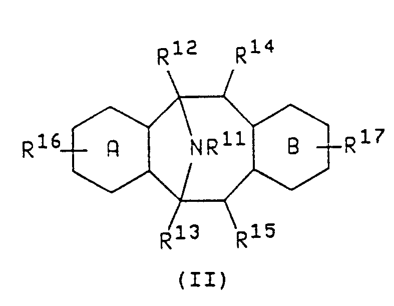 oder eines Salzes hiervon, worin die Ringe A und B unabhängig voneinander Cyclohexan-, Cyclohexen-, Cyclohexadien- oder Benzolringe bedeuten, R¹¹ die Bedeutung von R¹ im Anspruch 1 hat, und R¹² bis R¹⁷ unabhängig voneinander Wasserstoff, Hydroxy, C₁₋₆ Alkyl, Aryl, Arylalkyl, C₁₋₆ Alkoxy oder Halogen darstellen.

4. Eine Verbindung, wie sie im Anspruch 3 beansprucht wird, worin die Ringe A und B Benzolringe und R¹⁶ und R¹⁷ unabhängig voneinander Wasserstoff, Methyl, Hydroxy, Methoxy, Brom oder Chlor darstellen.

5. Eine Verbindung, wie sie im Anspruch 3 oder Anspruch 4 beansprucht wird, worin R¹³ Methyl darstellt.

6. Eine Verbindung, wie sie im Anspruch 1 beansprucht wird, ausgewählt aus:
6-Methyl-5,6,11,12-tetrahydro-dibenzo[a,e]cycloocten-6,11-imin;
11-n-Butyl-6-methyl-5,6,11,12-tetrahydro-dibenzo[a,e]cycloocten-6,11-imin;
5,6-Dimethyl-5,6,11,12-tetrahydro-dibenzo[a,e]cycloocten-6,11-imin;
2-Brom-6-methyl-5,6,11,12-tetrahydro-dibenzo[a,e]cycloocten-6,11-imin;
9-Brom-6-methyl-5,6,11,12-tetrahydro-dibenzo[a,e]cycloocten-6,11-imin;
3,6-Dimethyl-5,6,11,12-tetrahydro-dibenzo[a,e]cycloocten-6,11-imin;
6,8-Dimethyl-5,6,11,12-tetrahydro-dibenzo[a,e]cycloocten-6,11-imin;
2-Chlor-6-methyl-5,6,11,12-tetrahydro-dibenzo[a,e]cycloocten-6,11-imin;
9-Chlor-6-methyl-5,6,11,12-tetrahydro-dibenzo[a,e]cycloocten-6,11-imin;
3-Brom-6-methyl-5,6,11,12-tetrahydro-dibenzo[a,e]cycloocten-6,11-imin;
6-Methyl-5,6,6a-exo,7,8,9,10,10a-exo,11,12-decahydro-dibenzo[a,e]cycloocten-6,11-imin;
6-Methyl-5,6,6a-endo,7,8,9,10,10a-endo,11,12-decahydro-dibenzo[a,e]cycloocten-6,11-imin;
5,6,11,12-Tetrahydro-dibenzo[a,e]cycloocten-6,11-imin;
6,11-Dimethyl-5,6,11,12-tetrahydro-dibenzo[a,e]cycloocten-6,11-imin;
2-Methoxy-6-methyl-5,6,11,12-tetrahydro-dibenzo[a,e]cycloocten-6,11-imin;
3-Methoxy-6-methyl-5,6,11,12-tetrahydro-dibenzo[a,e]cycloocten-6,11-imin;
2-Hydroxy-6-methyl-5,6,11,12-tetrahydro-dibenzo[a,e]cycloocten-6,11-imin;
3-Hydroxy-6-methyl-5,6,11,12-tetrahydro-dibenzo[a,e]cycloocten-6,11-imin;
12-exo-Hydroxy-6-methyl-5,6,11,12-tetrahydro-dibenzo[a,e]cycloocten-6,11-imin;
1-Chlor-6-methyl-5,6,11,12-tetrahydro-dibenzo[a,e]cycloocten-6,11-imin;
10-Chlor-6-methyl-5,6,11,12-tetrahydro-dibenzo[a,e]cycloocten-6,11-imin;
6-Ethyl-5,6,11,12-tetrahydro-dibenzo[a,e]cycloocten-6,11-imin;
8-Brom-6-methyl-5,6,11,12-tetrahydro-dibenzo[a,e]cycloocten-6,11-imin;
11-Hydroxy-6-methyl-5,6,11,12-tetrahydro-dibenzo[a,e]cycloocten-6,11-imin;
6,13-Dimethyl-5,6,11,12-tetrahydro-dibenzo[a,e]cycloocten-6,11-imin;
und deren Salzen.

7. Eine pharmazeutische Zusammensetzung, umfassend eine Verbindung, wie sie in irgendeinem der vorhergehenden Ansprüche beansprucht wird, in Verbindung mit einem pharmazeutisch annehmbaren Träger oder Vehikel.

8. Eine Verbindung, wie sie in irgendeinem der Ansprüche 1 bis 6 beansprucht wird, zur Verwendung als therapeutisches Mittel.

9. Die Verwendung einer Verbindung, wie sie in irgendeinem der Ansprüche 1 bis 6 beansprucht wird, zur Herstellung eines Arzneimittels für die Behandlung und/oder Vorbeugung von Krämpfen und/oder neurodegenerativen Störungen.

10. Ein Verfahren zur Herstellung einer Verbindung, wie sie in irgendeinem der Ansprüche 1 bis 6 beansprucht wird, umfassend:
(A) zur Herstellung einer Verbindung der Formel I, in der R¹ eine andere Bedeutung aufweist als Hydroxy: Reduktion einer Verbindung der Formel III 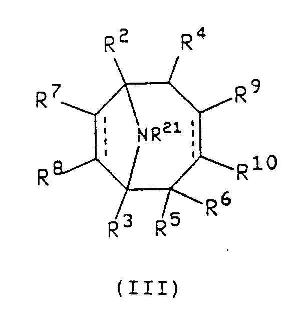 worin R²¹ Hydroxy oder C₁₋₆ Alkoxy darstellt, und R² bis R¹⁰ wie im Anspruch 1 definiert sind, und, falls R¹ eine andere Bedeutung als Wasserstoff besitzt, Alkylierung oder Alkenylierung des Produkts; oder
(B) zur Herstellung einer Verbindung der Formel I, in der R² Alkyl, Carboxymethyl oder Halogen darstellt:
Reaktion einer Verbindung der Formel VI 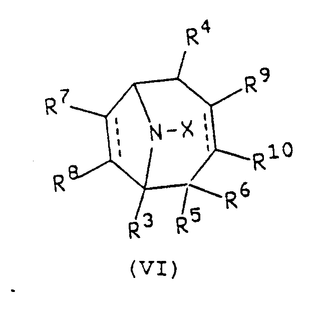 worin R³ bis R¹⁰ wie im Anspruch 1 definiert sind und X eine austretende Gruppe darstellt, mit einem Anion ⁻R₂; oder
(C) zur Herstellung einer Verbindung der Formel I, in der R² und/oder R³ Hydroxy darstellen:
Behandlung einer Verbindung der Formel III, wie sie oben definiert ist, in der R² und/oder R³ Wasserstoff bedeuten, mit Manganacetat, Reduktion des hierbei erhaltenen Produkts und, falls R¹ eine andere Bedeutung als Wasserstoff besitzt, Alkylierung oder Alkenylierung des erhaltenen Produkts; oder
(D) zur Herstellung einer Verbindung der Formel I, in der R⁴ und/oder R⁵ Hydroxy darstellen, wenn R⁶ Wasserstoff ist:
Reduktion der entsprechenden Oxo- oder Dioxoverbindung, in der das Stickstoffatom geschützt ist, Entfernung der Schutzgruppe vom Stickstoffatom, und gewünschtenfalls Auftrennung des hierbei erhaltenen Produkts in seine einzelnen endo- und exo-Isomere mittels konventioneller Methoden;
(E) zur Herstellung einer Verbindung der Formel I, in der R², R³, R⁴ und/oder R⁵ Fluor darstellen:
Behandlung der entsprechenden Hydroxy-Verbindung mit Diethylamino-schwefeltrifluorid; oder
(F) zur Herstellung einer Verbindung der Formel VII 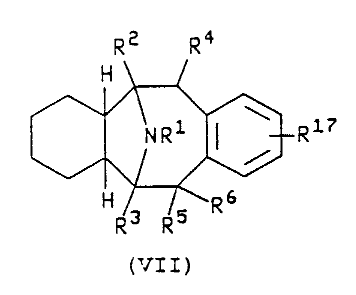 worin R¹ und R⁶ wie im Anspruch 1 definiert sind und R¹⁷ wie im Anspruch 3 definiert ist:
Reduktion der entsprechenden Dibenzoverbindung.

## Patentansprüche (Patentansprüche für folgende(n) Vertragsstaat(en): ES)

1. Ein Verfahren zur Herstellung einer Verbindung der Formel I 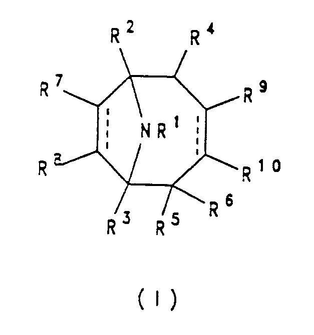 oder eines Salzes hiervon, worin
die gestrichelten Linien fakultative Doppelbindungen bedeuten;
R¹ Wasserstoff, Hydroxy, C₂₋₆ Alkenyl, Alkyl, enthaltend 1 bis 6 Kohlenstoffatome, Aminoalkyl, enthaltend 1 bis 6 Kohlenstoffatome,oder Hydroxyalkyl, enthaltend 1 bis 6 Kohlenstoffatome, bedeutet;
R², R³, R⁴, R⁵ und R⁶ unabhängig voneinander Wasserstoff, Hydroxy, Fluor, C₂₋₆ Alkenyl, Aryl, Alkyl, enthaltend 1 bis 6 Kohlenstoffatome, oder Alkyl, enthaltend 1 bis 6 Kohlenstoffatome, substituiert durch Aryl, Amino, Hydroxy, Carboxy oder Fluor, bedeuten; und
R⁷, R⁸, R⁹ und R¹⁰ unabhängig voneinander Wasserstoff, eine Kohlenwasserstoffgruppe mit bis zu 18 Kohlenstoffatomen, oder eine heterocyclische Gruppe bedeuten, wobei die Kohlenwasserstoffgruppen oder die heterocyclischen Gruppen gegebenenfalls durch eine Gruppe substituiert sind, die aus Halogen, C₁₋₆ Alkyl, Aryl, Arylalkyl, C₁₋₆ Alkoxy, Halogen(C₁₋₆)alkyl, Hydroxy, Amino, Nitro, Carboxy, C₁₋₆ Alkoxycarbonyl, C₁₋₆ Alkoxycarbonyl-(C₁₋₆)alkyl, C₁₋₆ Alkylcarbonyloxy und C₁₋₆ Alkylcarbonylgruppen ausgewählt ist, unter der Bedingung, daß R⁷, R⁸, R⁹ und R¹⁰ nicht gleichzeitig Wasserstoff sind; oder
R⁷ und R⁸ und/oder R⁹ und R¹⁰ können einen gesättigten oder ungesättigten C₄₋₉ Kohlenwasserstoffring oder heterocyclischen Ring komplettieren; welches Verfahren umfaßt:
(A) zur Herstellung einer Verbindung der Formel I, in der R¹ eine andere Bedeutung aufweist als Hydroxy:
Reduktion einer Verbindung der Formel III 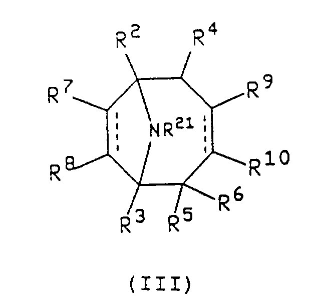 worin R²¹ Hydroxy oder C₁₋₆ Alkoxy darstellt, und R² bis R¹⁰ wie oben definiert sind, und, falls R¹ eine andere Bedeutung als Wasserstoff besitzt, Alkylierung oder Alkenylierung des Produkts; oder
(B) zur Herstellung einer Verbindung der Formel I, in der R² Alkyl, Carboxymethyl oder Halogen darstellt: Reaktion einer Verbindung der Formel VI 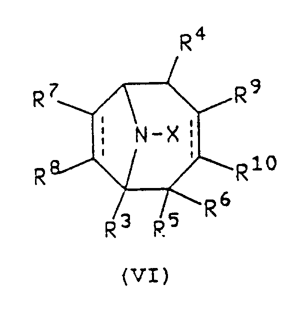 worin R³ bis R¹⁰ wie oben definiert sind und X eine austretende Gruppe darstellt, mit einem Anion ⁻R₂; oder
(C) zur Herstellung einer Verbindung der Formel I, in der R² und/oder R³ Hydroxy darstellen:
Behandlung einer Verbindung der Formel III, wie sie oben definiert ist, in der R² und/oder R³ Wasserstoff bedeuten, mit Manganacetat, Reduktion des hierbei erhaltenen Produkts und, falls R¹ eine andere Bedeutung als Wasserstoff besitzt, Alkylierung oder Alkenylierung des erhaltenen Produkts; oder
(D) zur Herstellung einer Verbindung der Formel I, in der R⁴ und/oder R⁵ Hydroxy darstellen, wenn R⁶ Wasserstoff ist:
Reduktion der entsprechenden Oxo- oder Dioxoverbindung, in der das Stickstoffatom geschützt ist, Entfernung der Schutzgruppe vom Stickstoffatom, und gewünschtenfalls Auftrennung des hierbei erhaltenen Produkts in seine einzelnen endo- und exo-Isomere mittels konventioneller Methoden;
(E) zur Herstellung einer Verbindung der Formel I, in der R², R³, R⁴ und/oder R⁵ Fluor darstellen:
Behandlung der entsprechenden Hydroxy-Verbindung mit Diethylamino-schwefeltrifluorid; oder
(F) zur Herstellung einer Verbindung der Formel VII 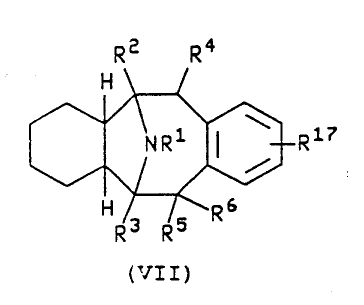 worin R¹ und R⁶ wie im Anspruch 1 definiert sind und R¹⁷ Wasserstoff, Hydroxy, C₁₋₆ Alkyl, Aryl, Arylalkyl, C₁₋₆ Alkoxy oderHalogen darstellen:
Reduktion der entsprechenden Dibenzoverbindung.

2. Ein Verfahren, wie es im Anspruch 1 beansprucht wird, zur Herstellung einer Verbindung, worin R² uns R⁶ unabhängig voneinander Wasserstoff, Hydroxy oder C₁₋₄ Alkyl bedeuten.

3. Ein Verfahren, wie es im Anspruch 1 beansprucht wird, zur Herstellung einer Verbindung der Formel II 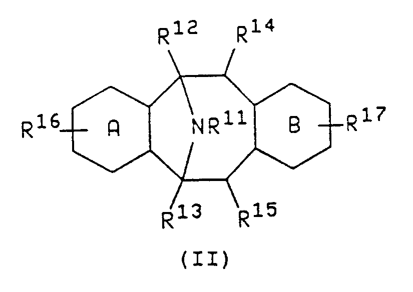 oder eines Salzes hiervon, worin die Ringe A und B unabhängig voneinander Cyclohexan-, Cyclohexen-, Cyclohexadien- oder Benzolringe bedeuten, R¹¹ die Bedeutung von R¹ im Anspruch 1 hat, und R¹² bis R¹⁷ unabhängig voneinander Wasserstoff, Hydroxy, C₁₋₆ Alkyl, Aryl, Arylalkyl, C₁₋₆ Alkoxy oder Halogen darstellen.

4. Ein Verfahren, wie es im Anspruch 3 beansprucht wird, zur Herstellung einer Verbindung, worin die Ringe A und B Benzolringe und R¹⁶ und R¹⁷ unabhängig voneinander Wasserstoff, Methyl, Hydroxy, Methoxy, Brom oder Chlor darstellen.

5. Ein Verfahren, wie es im Anspruch 3 oder im Anspruch 4 beansprucht wird, zur Herstellung einer Verbindung, worin R¹³ Methyl darstellt.

6. Ein Verfahren, wie es im Anspruch 1 beansprucht wird, zur Herstellung einer Verbindung ausgewählt aus:
6-Methyl-5,6,11,12-tetrahydro-dibenzo[a,e]cycloocten-6,11-imin;
11-n-Butyl-6-methyl-5,6,11,12-tetrahydro-dibenzo[a,e]cycloocten-6,11-imin;
5,6-Dimethyl-5,6,11,12-tetrahydro-dibenzo[a,e]cycloocten-6,11-imin;
2-Brom-6-methyl-5,6,11,12-tetrahydro-dibenzo[a,e]cycloocten-6,11-imin;
9-Brom-6-methyl-5,6,11,12-tetrahydro-dibenzo[a,e]cycloocten-6,11-imin;
3,6-Dimethyl-5,6,11,12-tetrahydro-dibenzo[a,e]cycloocten-6,11-imin;
6,8-Dimethyl-5,6,11,12-tetrahydro-dibenzo[a,e]cycloocten-6,11-imin;
2-Chlor-6-methyl-5,6,11,12-tetrahydro-dibenzo[a,e]cycloocten-6,11-imin;
9-Chlor-6-methyl-5,6,11,12-tetrahydro-dibenzo[a,e]cycloocten-6,11-imin;
3-Brom-6-methyl-5,6,11,12-tetrahydro-dibenzo[a,e]cycloocten-6,11-imin;
6-Methyl-5,6,6a-exo,7,8,9,10,10a-exo,11,12-decahydro-dibenzo[a,e]cycloocten-6,11-imin;
6-Methyl-5,6,6a-endo,7,8,9,10,10a-endo,11,12-decahydro-dibenzo[a,e]cycloocten-6,11-imin;
5,6,11,12-Tetrahydro-dibenzo[a,e]cycloocten-6,11-imin;
6,11-Dimethyl-5,6,11,12-tetrahydro-dibenzo[a,e]cycloocten-6,11-imin;
2-Methoxy-6-methyl-5,6,11,12-tetrahydro-dibenzo[a,e]cycloocten-6,11-imin;
3-Methoxy-6-methyl-5,6,11,12-tetrahydro-dibenzo[a,e]cycloocten-6,11-imin;
2-Hydroxy-6-methyl-5,6,11,12-tetrahydro-dibenzo[a,e]cycloocten-6,11-imin;
3-Hydroxy-6-methyl-5,6,11,12-tetrahydro-dibenzo[a,e]cycloocten-6,11-imin;
12-exo-Hydroxy-6-methyl-5,6,11,12-tetrahydro-dibenzo[a,e]cycloocten-6,11-imin;
1-Chlor-6-methyl-5,6,11,12-tetrahydro-dibenzo[a,e]cycloocten-6,11-imin;
10-Chlor-6-methyl-5,6,11,12-tetrahydro-dibenzo[a,e]cycloocten-6,11-imin;
6-Ethyl-5,6,11,12-tetrahydro-dibenzo[a,e]cycloocten-6,11-imin;
8-Brom-6-methyl-5,6,11,12-tetrahydro-dibenzo[a,e]cycloocten-6,11-imin;
11-Hydroxy-6-methyl-5,6,11,12-tetrahydro-dibenzo[a,e]cycloocten-6,11-imin;
6,13-Dimethyl-5,6,11,12-tetrahydro-dibenzo[a,e]cycloocten-6,11-imin;
und deren Salzen.

7. Ein Verfahren zur Herstellung einer pharmazeutischen Zusammensetzung, welches Mischen einer Verbindung, die hergestellt wird, wie es in irgendeinem der vorhergehenden Ansprüche beansprucht wird, mit einem pharmazeutisch annehmbaren Träger oder Vehikel umfaßt.

8. Die Verwendung einer Verbindung, die hergestellt wird, wie es in irgendeinem der Ansprüche 1 bis 6 beansprucht wird, zur Herstellung eines Arzneimittels für die Behandlung und/oder Vorbeugung von Krämpfen und/oder neurodegenerativen Störungen.

## Revendications (Revendications pour l'(les) Etat(s) contractant(s) suivant(s): AT, BE, CH, DE, FR, GB, IT, LI, LU, NL, SE)

1. Composé de formule I: 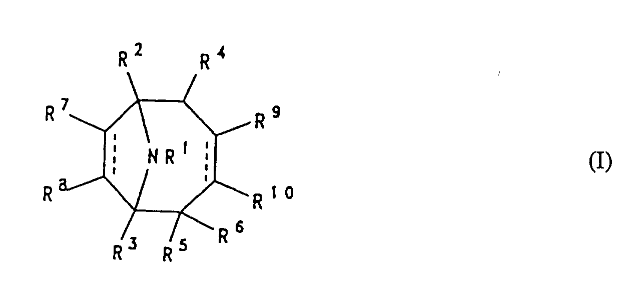 ou un de ses sels, dans laquelle:
Les traits en pointillés représentent des doubles liaisons facultatives;
R¹ représente un atome d'hydrogène ou un groupe hydroxy, alcényle en C₂₋₆, alkyle contenant de 1 à 6 atomes de carbone, aminoalkyle contenant de 1 à 6 atomes de carbone ou hydroxyalkyle contenant de 1 à 6 atomes de carbone;
R², R³, R⁴, R⁵ et R⁶ représentent indépendamment un atome d'hydrogène ou de fluor ou un groupe hydroxy, alcényle en C₂₋₆, aryle, alkyle contenant de 1 à 6 atomes de carbone ou alkyle contenant de 1 à 6 atomes de carbone à substituant(s) aryle, amino, hydroxy, carboxy ou fluoro ; et
R^{7,} R⁸, R⁹ et R¹⁰ représentent indépendamment un atome d'hydrogène ou un groupe hydrocarboné ayant jusqu'à 18 atomes de carbone ou un groupe hétérocyclique, ces groupes hydrocarbonés ou hétérocycliques étant éventuellement substitués par un groupe choisi parmi les atomes d'halogène et les groupes alkyle en C₁₋₆, aryle, arylalkyle, alcoxy en C₁₋₆, halogénoalkyle en C₁₋₆, hydroxy, amino, nitro, carboxy, (alcoxy en C₁₋₆)carbonyle, (alcoxy en C₁₋₆)carbonyl-(alkyle en C₁₋₆), (alkyle en C₁₋₆)carbonyloxy et (alkyle en C₁₋₆)carbonyle à condition que R^{7,} R⁸, R⁹ et R¹⁰ ne soient pas simultanément un atome d'hydrogène; ou
R ⁷ ^{et} R⁸ et/ou R⁹ et R¹⁰ peuvent compléter un noyau hétérocyclique ou hydrocarboné en C₄₋₉, saturé ou insaturé.

2. Composé selon la revendication 1, dans lequel R² et R⁶ représentent indépendamment un atome d'hydrogène ou un groupe hydroxy ou alkyle en C₁₋₄.

3. Composé de formule II : 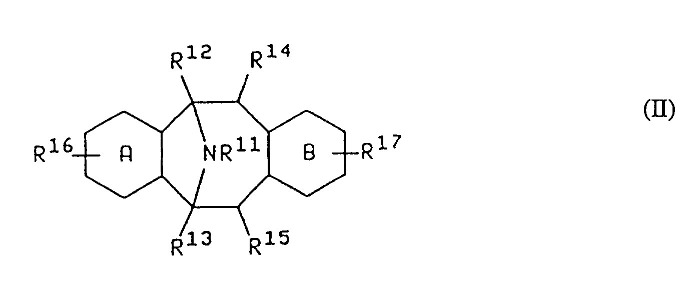 ou un de ses sels, dans laquelle les noyaux A et B représentent indépendamment des noyaux cyclohexane, cyclohexène, cyclohexadiène ou benzène ; R¹¹ est tel que défini dans la revendication 1 pour R¹ ; et R¹² à R¹⁷ représentent indépendamment un atome d'hydrogène ou d'halogène ou un groupe hydroxy, alkyle en C₁₋₆, aryle, arylalkyle ou alcoxy en C₁₋₆.

4. Composé selon la revendication 3, dans lequel les noyaux A et B représentent des noyaux benzèniques et R¹⁶ et R¹⁷ représentent indépendamment un atome d'hydrogène, de brome ou de chlore, ou un groupe méthyle, hydroxy ou méthoxy.

5. Composé selon la revendication 3 ou 4, dans lequel R¹³ représente un groupe méthyle.

6. Composé selon la revendication 1, choisi parmi les suivants :
· 6-méthyl-5,6,11,12-tétrahydrodibenzo(a,e)cyclooctèn-6,11-imine ;
· 11-n-butyl-6-méthyl-5,6,11,12-tétrahydrodibenzo(a,e)cyclooctèn-6,11-imine ;
· 5,6-diméthyl-5,6,11,12-tétrahydrodibenzo(a,e)cyclooctèn-6,11-imine ;
· 2-bromo-6-méthyl-5,6,11,12-tétrahydrodibenzo(a,e)cyclooctèn-6,11-imine ;
· 9-bromo-6-méthyl-5,6,11,12-tétrahydrodibenzo(a,e)cyclooctèn-6,11-imine ;
· 3,6-diméthyl-5,6,11,12-tétrahydrodibenzo(a,e)cyclooctèn-6,11-imine ;
· 6,8-diméthyl-5,6,11,12-tétrahydrodibenzo(a,e)cyclooctèn-6,11-imine ;
· 2-chloro-6-méthyl-5,6,11,12-tétrahydrodibenzo(a,e)cyclooctèn-6,11-imine ;
· 9-chloro-6-méthyl-5,6,11,12-tétrahydrodibenzo(a,e)cyclooctèn-6,11-imine ;
· 3-bromo-6-méthyl-5,6,11,12-tétrahydrodibenzo(a,e)cyclooctèn-6,11-imine ;
· 6-méthyl-5,6,6a-exo-7,8,9,10,10a-exo,11,12-décahydrodibenzo(a,e)cyclooctèn-6,11-imine ;
· 6-méthyl-5,6,6a-endo,7,8,9,10,10a-endo,11,12-décahydrodibenzo(a,e)cyclooctèn-6,11-imine ;
· 5,6,11,12-tétrahydrodibenzo(a,e)cyclooctèn-6,11-imine ;
· 6,11-diméthyl-5,6,11,12-tétrahydrodibenzo(a,e)cyclooctèn-6,11-imine ;
· 2-méthoxy-6-méthyl-5,6,11,12-tétrahydrodibenzo(a,e)cyclooctèn-6,11-imine ;
· 3-méthoxy-6-méthyl-5,6,11,12-tétrahydrodibenzo(a,e)cyclooctèn-6,11-imine ;
· 2-hydroxy-6-méthyl-5,6,11,12-tétrahydrodibenzo(a,e)cyclooctèn-6,11-imine ;
· 3-hydroxy-6-méthyl-5,6,11,12-tétrahydrodibenzo(a,e)cyclooctèn-6,11-imine ;
· 12-exo-hydroxy-6-méthyl-5,6,11,12-tétrahydrodibenzo(a,e)cyclooctèn-6,11-imine ;
· 1-chloro-6-méthyl-5,6,11,12-tétrahydrodibenzo(a,e)cyclooctèn-6,11-imine ;
· 10-chloro-6-méthyl-5,6,11,12-tétrahydrodibenzo(a,e)cyclooctèn-6,11-imine ;
· 6-éthyl-5,6,11,12-tétrahydrodibenzo(a,e)cyclooctèn-6,11-imine ;
· 8-bromo-6-méthyl-5,6,11,12-tétrahydrodibenzo(a,e)cyclooctèn-6,11-imine ;
· 11-hydroxy-6-méthyl-5,6,11,12-tétrahydrodibenzo(a,e)cyclooctèn-6,11-imine ;
· 6,13-diméthyl-5,6,11,12-tétrahydrodibenzo(a,e)cyclooctèn-6,11-imine ;
· et leurs sels.

7. Composition pharmaceutique comprenant un composé selon l'une quelconque des revendications précédentes, en association avec un excipient ou véhicule pharmaceutiquement acceptable.

8. Composé selon l'une quelconque des revendications 1 à 6, à utiliser comme agent thérapeutique.

9. Utilisation d'un composé selon l'une queleonque des revendications 1 à 6, pour la préparation d'un médicament pour le traitement et/ou la prévention des convulsions et/ou des troubles neuro-dégénératifs.

10. Procédé pour la préparation d'un composé selon l'une quelconque des revendications 1 à 6, qui comprend :
(A) pour la préparation d'un composé de formule I dans laquelle
R¹ est différent d'un groupe hydroxy :
la réduction d'un composé de formule III : 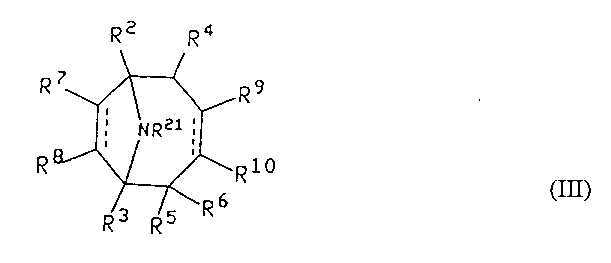 dans laquelle R²¹ représente un groupe hydroxy ou alcoxy en C₁₋₆, et R² à R¹⁰ sont tels que définis dans la revendication 1 ; et lorsque R¹ est différent d'un atome d'hydrogène, l'alkylation ou l'alcénylation du produit;
ou
(B) pour la préparation d'un composé de formule I dans laquelle R² représente un groupe alkyle, carboxyméthyle ou un atome d'halogène :
la réaction d'un composé de formule VI 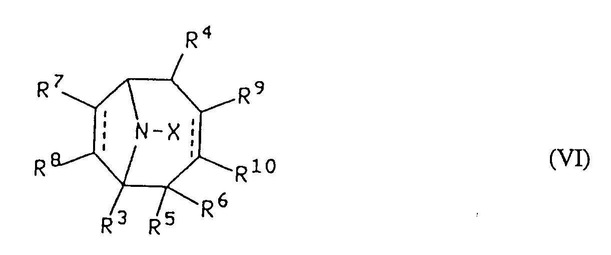 dans laquelle R³ à R¹⁰ sont tels que définis dans la revendication 1 et X représente un groupe partant ; avec un anion ⁻R² ; ou
(C) pour la préparation d'un composé de formule I dans laquelle R² et/ou R³ représentent un groupe hydroxy ;
le traitement d'un composé de formule III tel que défini ci-dessus, dans laquelle R² et/ou R³ représentent un atome d'hydrogène, avec l'acétate de manganèse ; la réduction du produit ainsi obtenu ; et lorsque R¹ est différent d'un atome d'hydrogène, l'alkylation ou l'alcénylation du produit résultant ; ou
(D) pour la préparation d'un composé de formule I dans laquelle R⁴ et/ou R⁵ représentent un groupe hydroxy lorsque R⁶ représente un atome d'hydrogène :
la réduction du composé oxo ou dioxo correspondant, dans lequel l'atome d'azote est protégé ; la déprotection de l'atome d'azote ; et si nécessaire la séparation du produit ainsi obtenu en ses isomères endo et exo individuels par des techniques classiques ; ou
(E) pour la préparation d'un composé de formule I, dans laquelle R², R³, R⁴ et/ou R⁵ représentent un atome de fluor :
le traitement du composé hydroxy correspondant avec le trifluorure de diéthylaminosoufre ; ou
(F) pour la préparation d'un composé de formule VII : 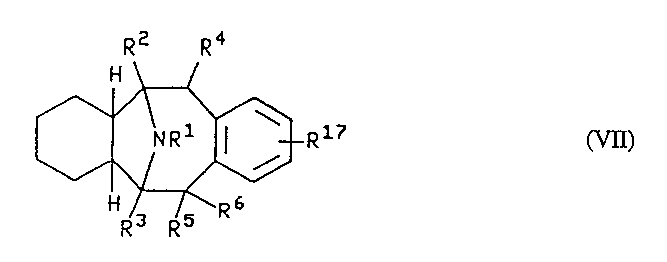 dans laquelle R¹ et R⁶ sont tels que définis dans la revendication 1 et R¹⁷ est tel que défini dans la revendication 3 :
la réduction du composé dibenzo correspondant.

## Revendications (Revendications pour l'(les) Etat(s) contractant(s) suivant(s): ES)

1. Procédé pour la préparation d'un composé de formule I : 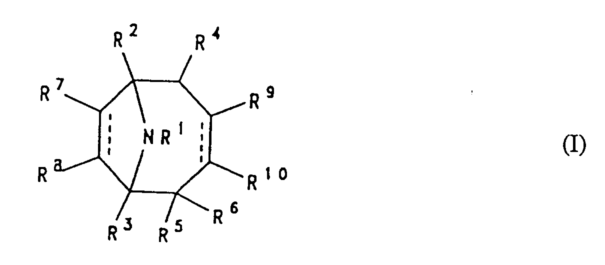 ou d'un de ses sels, dans laquelle :
· les traits en pointillés représentent des doubles liaisons facultatives ;
· R¹ représente un atome d'hydrogène ou un groupe hydroxy, alcényle en C₂₋₆, alkyle contenant de 1 à 6 atomes de carbone, aminoalkyle contenant de 1 à 6 atomes de carbone ou hydroxyalkyle contenant de 1 à 6 atomes de carbone ;
· R², R³, R⁴, R⁵ et R⁶ représentent indépendamment un atome d'hydrogène ou de fluor ou un groupe hydroxy, alcényle en C₂₋₆, aryle, alkyle contenant de 1 à 6 atomes de carbone ou alkyle contenant de 1 à 6 atomes de carbone à substituant(s) aryle, amino, hydroxy, carboxy ou fluoro ; et
R⁷, R⁸, R⁹ et R¹⁰ représentent indépendamment un atome d'hydrogène ou un groupe hydrocarboné ayant jusqu'à 18 atomes de carbone ou un groupe hétérocyclique, ces groupes hydrocarbonés ou hétérocycliques étant éventuellement substitués par un groupe choisi parmi les atomes d'halogène et les groupes alkyle en C₁₋₆, aryle, arylalkyle, alcoxy en C₁₋₆, halogénoalkyle en C₁₋₆, hydroxy, amino, nit:ro, carboxy, (alcoxy en C₁₋₆)carbonyle, (alcoxy en C₁₋₆)carbonyl-(alkyle en C₁₋₆), (alkyle en C₁₋₆)carbonyloxy et (alkyle en C₁₋₆)carbonyle à condition que R⁷, R⁸, R⁹ et R¹⁰ ne soient pas simultanément un atome d'hydrogène ; ou
R⁷ et R⁸ et/ou R⁹ et R¹⁰ peuvent compléter un noyau hétérocyclique ou hydrocarboné en C₄₋₉, saturé ou insaturé ;
qui comprend :
(A) pour la préparation d'un composé de formule I dans laquelle
R¹ est différent d'un groupe hydroxy :
la réduction d'un composé de formule III : 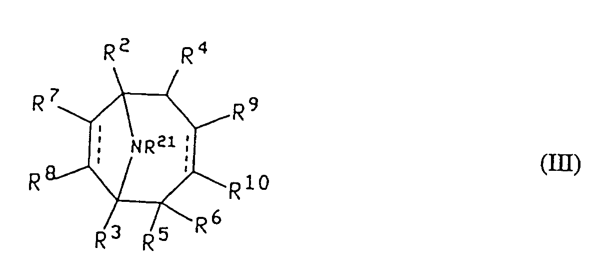 dans laquelle R²¹ représente un groupe hydroxy ou alcoxy en C₁₋₆, et R² à R¹⁰ sont tels que définis dans la revendication 1 ; et lorsque R¹ est différent d'un atome d'hydrogène, l'alkylation ou l'alcénylation du produit ;
ou
(B) pour la préparation d'un composé de formule I dans laquelle R² représente un groupe alkyle, carboxynéthyle ou un atome d'halogène :
la réaction d'un composé de formule VI 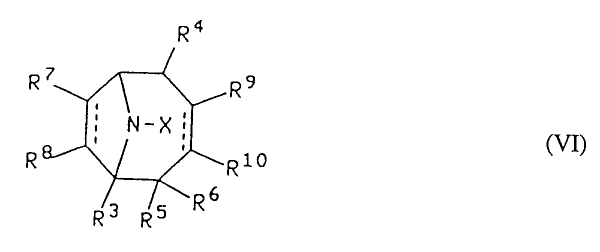 dans laquelle R³ à R¹⁰ sont tels que définis ci-dessus, et X représente un groupe partant ; avec un anion ⁻R² ; ou
(C) pour la préparation d'un composé de formule I dans laquelle R² et/ou R³ représentent un groupe hydroxy ;
le traitement d'un composé de formule III tel que défini ci-dessus, dans laquelle R² et/ou R³ représentent un atome d'hydrogène, avec l'acétate de manganèse ; la réduction du produit ainsi obtenu ; et lorsque R¹ est différent d'un atome d'hydrogène, l'alkylation ou l'alcénylation du produit résultant ; ou
(D) pour la préparation d'un composé de formule I dans laquelle R⁴ et/ou R⁵ représentent un groupe hydroxy lorsque R⁶ représente un atome d'hydrogène :
la réduction du composé oxo ou dioxo correspondant, dans lequel l'atome d'azote est protégé ; la déprotection de l'atome d'azote ; et si nécessaire la séparation du produit ainsi obtenu en ses isomères endo et exo individuels par des techniques classiques ; ou
(E) pour la préparation d'un composé de formule I, dans laquelle R², R³, R⁴ et/ou R⁵ représentent un atome de fluor :
le traitement du composé hydroxy correspondant avec le trifluorure de diéthylaminosoufre; ou
(F) pour la préparation d'un composé de formule VII : 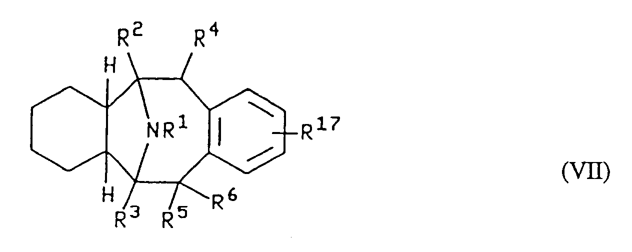 dans laquelle R¹ et R⁶ sont tels que définis dans la revendication 1 et R¹⁷ représente un atome d'hydrogène ou d'halogéne ou un groupe alkyle en C₁₋₆, aryle, arylalkyle ou alcoxy en C₁₋₆:
la réduction du composé dibenzo correspondant.

2. Procédé selon la revendication 1 pour la préparation d'un composé dans lequel R² et R⁶ représentent indépendamment un atome d'hydrogène ou un groupe hydroxy ou alkyle en C₁₋₄.

3. Procédé selon la revendication 1, pour la préparation d'un composé de formule II : 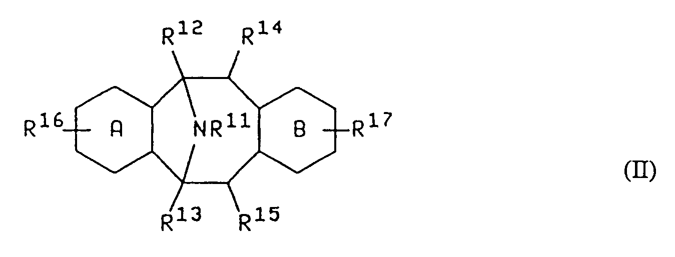 ou d'un de ses sels, dans laquelle les noyaux A et B représentent indépendamment des noyaux cyclohexane, cyclohexène, cyclohexadiène ou benzène ; R¹¹ est tel que défini dans la revendication 1 pour R¹ ; et R¹² à R¹⁷ représentent indépendamment des atomes d'hydrogène ou d'halogène ou des groupes hydroxy, alkyle en C₁₋₆, aryle, arylalkyle ou alcoxy en C₁₋₆.

4. Procédé selon la revendication 3, pour la préparation d'un composé dans lequel les noyaux A et B représentent des noyaux benzèniques et R¹⁶ et R¹⁷ représentent indépendamment un atome d'hydrogène, de brome ou de chlore, ou un groupe méthyle, hydroxy ou méthoxy.

5. Procédé selon la revendication 3 ou la revendication 4, pour la préparation d'un composé dans lequel R¹³ représente un groupe méthyle.

6. Procédé selon la revendication 1, pour la préparation d'un composé choisi parmi les suivants :
· 6-méthyl-5,6,11,12-tétrahydrodibenzo(a,e)cyclooctèn-6,11-imine ;
· 11-n-butyl-6-méthyl-5,6,11,12-tétrahydrodibenzo(a,e)cyclooctèn-6,11-imine ;
· 5,6-diméthyl-5,6,11,12-tétrahydrodibenzo(a,e)cyclooctèn-6,11-imine ;
· 2-bromo-6-méthyl-5,6,11,12-tétrahydrodibenzo(a,e)cyclooctèn-6,11-imine ;
· 9-bromo-6-méthyl-5,6,11,12-tétrahydrodibenzo(a,e)cyclooctèn-6,11-imine ;
· 3,6-diméthyl-5,6,11,12-tétrahydrodibenzo(a,e)cyclooctèn-6,11-imine ;
· 6,8-diméthyl-5,6,11,12-tétrahydrodibenzo(a,e)cyclooctèn-6,11-imine ;
· 2-chloro-6-méthyl-5,6,11,12-tétrahydrodibenzo(ave)cyclooctèn-6,11-imine ;
· 9-chloro-6-méthyl-5,6,11,12-tétrahydrodibenzo(a,e)cyclooctèn-6,11-imine ;
· 3-bromo-6-méthyl-5,6,11,12-tétrahydrodibenzo(a,e)cyclooctèn-6,11-imine ;
· 6-méthyl-5,6,6a-exo-7,8,9,10,10a-exo,11,12-décahydrodibenzo(a,e)cyclooctèn-6,11-imine ;
· 6-méthyl-5,6,6a-endo,7,8,9,10,10a-endo,11,12-décahydrodibenzo(a,e) cyclooctèn,6,11-imine ;
· 5,6,11,12-tétrahydrodibenzo(a,e)cyclooctèn-6,11-imine ;
· 6,11-diméthyl-5,6,11,12-tétrahydrodibenzo(a,e)cyclooctèn-6,11-imine ;
· 2-méthoxy-6-méthyl-5,6,11,12-tétrahydrodibenzo(a,e)cyclooctèn-6,11-imine ;
· 3-méthoxy-6-méthyl-5,6,11,12-tétrahydrodibenzo(a,e)cyclooctèn-6,11-imine ;
· 2-hydroxy-6-méthyl-5,6,11,12-tétrahydrodibenzo(a,e)cyclooctèn-6,11-imine ;
· 3-hydroxy-6-méthyl-5,6,11,12-tétrahydrodibenzo(a,e)cyclooctèn-6,11-imine ;
· 12-exo-hydroxy-6-méthyl-5,6,11,12-tétrahydrodibenzo(a,e)cyclooctèn-6,11-imine ;
· 1-chloro-6-méthyl-5,6,11,12-tétrahydrodibenzo(a,e)cyclooctèn-6,11-imine ;
· 10-chloro-6-méthyl-5,6,11,12-tétrahydrodibenzo(a,e)cyclooctèn-6,11-imine ;
· 6-éthyl-5,6,11,12-tétrahydrodibenzo(a,e)cyclooctèn-6,11-imine ;
· 8-bromo-6-méthyl-5,6,11,12-tétrahydrodibenzo(a,e)cyclooctèn-6,11-imine ;
· 11-hydroxy-6-méthyl-5,6,11,12-tétrahydrodibenzo(a,e)cyclooctèn-6,11-imine ;
· 6,13-diméthyl-5,6,11,12-tétrahydrodibenzo(a,e)cyclooctèn-6,11-imine ;
· et leurs sels.

7. Procédé pour la préparation d'une composition pharmaceutique qui comprend le mélange d'un composé préparé selon l'une quelconque des revendications précédentes, avec un excipient ou véhicule pharmaceutiquement acceptable.

8. Utilisation d'un composé préparé selon l'une quelconque des revendications 1 à 6, pour la préparation d'un médicament pour le traitement et/ou la prévention de convulsions et/ou de troubles dégénératifs.
